# EUROPEAN PATENT APPLICATION

(11) **EP 4 570 257 A1**
(43) Date of publication of application: **18.06.2025**
(21) Application number: 24218839.9
(22) Date of filing: 10.12.2024
(51) Int. Cl.: A61K 35/19, A61K 9/19, A61P 39/00, A61M 1/00, C12N 5/00

(54) **USE OF A PURIFIED PLATELET-DERIVED EXOSOME DRY POWDER FOR RELIEVING INFLAMMATION OR INJURY**

(30) Priority: 11.12.2023 US 202363608323 P
(71) Applicant: Spirit Scientific Co., Ltd., New Taipei City 22175 (TW); Lin, Dao Lung, Steven, New Taipei City 22175 (TW)
(72) Inventor: CHEN, Chin-Ho, 22175 New Taipei City (TW); LIN, Dao Lung, 22175 New Taipei City (TW)
(74) Representative: Haseltine Lake Kempner LLP

(57) **Abstract**

The present invention provides a use of a purified platelet-derived exosome dry powder for relieving inflammation or injury, wherein the number of platelet-derived exosomes in each gram of the purified platelet-derived exosome dry powder is more than or equal to 1×10¹⁰.

## Description

### [FIELD OF THE INVENTION]

The present invention relates to a purified platelet-derived exosome dry powder, more specifically to the use of a purified platelet-derived exosome dry powder for relieving inflammation or injury.

### [BACKGROUND OF THE INVENTION]

Inflammation is a defense mechanism of the human body. Inflammatory reactions are often triggered in various body parts due to infection, trauma, or hypersensitivity to eliminate harmful stimuli, remove pathogens, and facilitate tissue repair. Inflammation can be categorized into acute and chronic inflammation such as dermatitis, allergic rhinitis, pneumonia, hepatitis, and enteritis. Dermatitis can be classified into irritant contact dermatitis (ICD) and allergic contact dermatitis; severe pneumonia can cause diseases such as chronic obstructive pulmonary disease (COPD) and pulmonary fibrosis while severe hepatitis can cause diseases such as liver cirrhosis and cancer.

Fibroblasts play an important role in the repair process of various tissues throughout the body. A series of wound repair responses occur during the repair process after tissue damage (usually accompanied by inflammatory reactions), including cell proliferation, cell migration, fibrogenesis, resolution, and remodeling (Reference 1). Taking skin wounds as an example, fibroblasts rapidly migrate to the provisional matrix and proliferate quickly, leading to fibrogenesis in the injured area. Moreover, fibroblasts are the primary source of extracellular matrix (ECM) proteins, mainly including collagen and fibronectin, which can form granulation tissue and subsequently provide structural integrity to the wound.

Exosomes are nanoscale extracellular vesicles (EVs) approximately 30-200 nanometers in diameter. They are secreted by most eukaryotic cells and contain bioactive molecules such as proteins, ribonucleic acids, deoxyribonucleic acids, microRNAs, and metabolites. Therefore, exosomes play an important role in intercellular communication, transmitting different information between cells through the bioactive molecules they carry (References 2, 3, 4). Exosomes can be found in a variety of biological fluids such as plasma, urine, semen, saliva, bronchial fluid, cerebral spinal fluid (CSF), breast milk, serum, amniotic fluid, synovial fluid, tears, lymph, bile, and gastric acid (Reference 3). Among these, plasma and serum contain white blood cell-derived exosomes, red blood cell-derived exosomes, and exosomes derived from other cells.

Current studies have shown that exosomes derived from certain cells or tissues can achieve tissue regeneration and repair by inhibiting inflammatory responses, promoting proliferation, suppressing apoptosis, and facilitating vascular proliferation. However, clinical studies have found that exosomes are prone to rupture, have unstable quality, and are difficult to preserve during the isolation and purification process, thus affecting their therapeutic efficacy.

There is an urgent market need for a pharmaceutical composition capable of overcoming current technological limitations to relieve or treat inflammation or injury in various parts.

### [SUMMARY OF THE INVENTION]

In view of the deficiencies from the prior art, the object of the present invention is to provide a purified platelet-derived exosome dry powder that can effectively relieve or treat the degree of inflammation or injury.

Another object of the present invention is to provide a method for preparing the purified platelet-derived exosome dry powder.

A further object of the present invention is to provide a use of the purified platelet-derived exosome dry powder in alleviating or treating the degree of inflammation or injury.

A further object of the present invention is to provide a use for preparing a pharmaceutical composition or health composition for use in relieving or treating the degree of inflammation or injury.

The present invention provides a method for preparing purified platelet-derived exosome dry powder, comprising: (a) taking one unit of a solution derived from blood and containing platelets, and performing a process of purifying platelets to obtain a pure platelet solution; wherein step (a) comprises: (a1) taking one unit of a solution derived from blood and containing platelets; (a2) performing a first sedimentation process to separate the solution derived from blood and containing platelets into a plasma layer and a blood cell layer; (a3) removing the blood cell layer to obtain a plasma layer solution; (a4) performing a second sedimentation process to settle platelets into a pellet; (a5) removing a supernatant and mixing the pellet with a solvent to obtain the pure platelet solution; (b) performing an activation procedure to activate platelets in the pure platelet solution and release exosomes to obtain an activated pure platelet solution; wherein the activation procedure is a freezing and then thawing procedure; (c) performing a purification procedure, comprising: (c1) performing a centrifugation procedure to remove most platelet-associated structures; (c2) performing a filter membranes filtration process to completely remove a platelet-associated structure pellet and obtain a purified platelet-derived exosome solution; (d) subjecting the purified platelet-derived exosome solution to a drying process to obtain a purified platelet-derived exosome dry powder containing a plurality of platelet-derived exosomes.

In some embodiments, the drying process is a freeze-drying process.

In some embodiments, step (a3) comprises a process of romoving white blood cells so that the number of white blood cells in the pure platelet solution is less than 10⁶ per milliliter (mL).

In some embodiments, step (a5) comprises a process of removing plasma so that the albumin concentration in the pure platelet solution is less than 3 g/dL; or step (a5) comprises a process of removing plasma so that the globulin concentration in the pure platelet solution is less than 2 g/dL; or step (a5) comprises a process of removing plasma so that the fibrinogen concentration in the pure platelet solution is less than 100 µg/mL.

In some embodiments, step (a5) comprises a process of removing plasma so that the albumin concentration in the pure platelet solution is less than 0.25, 0.5, 0.75, 1, 1.25, 1.5, 1.75, 2, 2.25, 2.5, or 2.75 g/dL.

In some embodiments, step (a5) comprises a process of removing plasma so that the globulin concentration in the pure platelet solution is less than 0.25, 0.5, 0.75, 1, 1.25, 1.5, or 1.75 g/dL.

In some embodiments, step (a5) comprises a process of removing plasma so that the fibrinogen concentration in the pure platelet solution is less than 10, 20, 30, 40, 50, 60, 70, 80, or 90 µg/mL.

In some embodiments, the solution derived from blood and containing platelets is whole blood, and the blood cell layer includes a buffy coat layer and a red blood cells layer.

In some embodiments, the freezing and then thawing procedure (ie, the freeze-thaw procedure) comprises: (b1') placing a container holding the pure platelet solution into an environment at -70°C ~ -196°C to freeze the pure platelet solution; (b2') raising the temperature of the container to thaw the frozen pure platelet solution, and performing the steps (b1') and (b2') 1, 2, 3, 4, 5, or more than 5 times.

In some embodiments, the freezing and then thawing procedure (i.e., freeze-thaw procedure) comprises: (b1) placing a container holding the pure platelet solution into liquid nitrogen (-196°C) to freeze the pure platelet solution; (b2) raising the temperature of the container to thaw the frozen pure platelet solution, and performing the steps (b1) and (b2) 1, 2, 3, 4, 5, or more than 5 times.

In some embodiments, the freezing and then thawing procedure (freeze-thaw procedure) comprises: (b1') placing a container holding the pure platelet solution into an environment at -80°C to freeze the pure platelet solution; (b2') raising the temperature of the container to thaw the frozen pure platelet solution, and performing the steps (b1') and (b2') 1, 2, 3, 4, 5, or more than 5 times.

In some embodiments, the freeze-drying process comprises lowering a temperature to less than or equal to -35°C and lowering a pressure to less than or equal to 80 mTorr.

In some embodiments, the freeze-drying process comprises lowering the temperature to less than or equal to -40°C, -45°C, -50°C, -60°C, -65°C, or -70°C.

In some embodiments, the freeze-drying process comprises lowering the pressure to less than or equal to 70, 60, 50, 40, or 30 mTorr.

In some embodiments, the solution derived from blood and containing platelets is whole blood, apheresis platelets, leukocyte-reduced apheresis platelets, or platelet-rich plasma (PRP), or any combination thereof; alternatively, the solution derived from blood and containing platelets is derived from an autologous blood sample, an allogeneic blood sample or mammalian blood sample, or a combination thereof.

In some embodiments, in step (a5), the solvent is sterile saline, water for injection, 0.45% NaCl, 4.5% hypertonic saline, sterile warm spring water, or isotonic saline; alternatively, in step (a5), a platelet concentration in the pure platelet solution is 1×10⁹-10×10⁹ platelets/mL.

In some embodiments, in step (a5), the platelet concentration in the pure platelet solution is 2×10⁹, 3×10⁹, 4×10⁹, 5×10⁹, 6×10⁹, 7×10⁹, 8×10⁹, or 9×10⁹ platelets/mL.

In some embodiments, the membrane filtration procedure in step (c2) uses a filter membrane with a pore size less than or equal to 0.45 µm to obtain a solution enriched in purified platelet-derived exosomes.

In some embodiments, the membrane filtration procedure in step (c2) uses a filter membrane with a pore size less than or equal to 0.22 µm to obtain a solution enriched in purified platelet-derived exosomes.

The invention further provides a purified platelet-derived exosome dry powder, which is prepared by a preparation method comprises: (a) taking one unit of a solution derived from blood and containing platelets, and performing a process of purifying platelets to obtain a pure platelet solution, wherein step (a) comprises: (a1) taking one unit of a solution derived from blood and containing platelets; (a2) performing a first sedimentation process to separate the solution into a plasma layer and a blood cell layer; (a3) removing the blood cell layer to obtain a plasma layer solution; (a4) performing a second sedimentation process to settle the platelets into a pellet; (a5) removing a supernatant and mixing the pellet with a solvent to obtain the pure platelet solution; (b) performing an activation procedure to activate platelets in the pure platelet solution and release exosomes to obtain an activated pure platelet solution, wherein the activation procedure is a freezing and then thawing procedure; (c) performing a purification procedure comprising : (c1) performing a sedimentation process to remove most platelet-associated structures; (c2) performing a filter membrane filtration process to completely remove a platelet-associated structure pellet and obtain a purified platelet-derived exosome solution; (d) subjecting the purified platelet-derived exosome solution to a drying process on to obtain a purified platelet-derived exosome dry powder containing a plurality of platelet-derived exosomes.

In some embodiments, the drying process is a freeze-drying process.

In some embodiments, the freezing and then thawing procedure (freeze-thaw procedure) comprises: (b1') placing a container holding the pure platelet solution into an environment at -80°C to freeze the pure platelet solution; (b2') raising the temperature of the container to thaw the frozen pure platelet solution, and performing the steps (b1') and (b2') 1, 2, 3, 4, 5, or more than 5 times.

In some embodiments, in the purified platelet-derived exosome dry powder a particle diameter of the platelet-derived exosomes is 20-150 nanometers, and the platelet-derived exosomes contain a exosome marker and a platelet marker; wherein the exosome marker is CD9, CD63, or CD81, or any combination thereof, and the platelet marker is at least one of CD41 and CD42b, or any combination thereof; wherein (1) each gram of purified platelet-derived exosome dry powder comprises at least 1×10¹⁰ platelet-derived exosomes; or (2) each gram of purified platelet-derived exosome dry powder comprises 1×10¹¹-1×10¹⁵ platelet-derived exosomes; or (3) each gram of purified platelet-derived exosome dry powder comprises 1×10¹², 2×10¹², 3×10¹², 4×10¹², 5×10¹², 6×10¹², 7×10¹², 8×10¹², 9×10¹², 1×10¹³, 1.1×10¹³, 1.2×10¹³, 1.3×10¹³, 1.4×10¹³, 1.5×10¹³, 1.6×10¹³, 1.7×10¹³, 1.8×10¹³, 1.9×10¹³, 2×10¹³, 2.5×10¹³, 3×10¹³, 3.5×10¹³, 4×10¹³, 4.5×10¹³, 5×10¹³, 5.5×10¹³, 6×10¹³, 6.5×10¹³, 7×10¹³, 7.5×10¹³, 8×10¹³, 8.5×10¹³, 9×10¹³, 9.5×10¹³, 1×10¹⁴, 1.5×10¹⁴, 2×10¹⁴, 2.5×10¹⁴, 3×10¹⁴, 3.5×10¹⁴, 4×10¹⁴, 4.5×10¹⁴, 5×10¹⁴, 5.5×10¹⁴, 6×10¹⁴, 6.5×10¹⁴, 7×10¹⁴, 7.5×10¹⁴, 8×10¹⁴, 8.5×10¹⁴, 9×10¹⁴, or 9.5×10¹⁴ platelet-derived exosomes.

In some embodiments, (1) each gram of purified platelet-derived exosome dry powder comprises at least 50 µg of microRNA; or (2) a content of microRNA in each gram of purified platelet-derived exosome dry powder is 100 µg-2, 500 µg.

In some embodiments: (1) a content of PDGF-BB (platelet-derived growth factor-BB) in each gram of purified platelet-derived exosome dry powder is 0.01 ng-2,000 ng; or (2) a content of VEGF (vascular endothelial growth factor) in each gram of purified platelet-derived exosome dry powder is 50 pg-100,000 pg; or (3) a content of IGF (insulin-like growth factor) in each gram of purified platelet-derived exosome dry powder is 1 pg-3,000 pg; or (4) a content of TGF-β1 (transforming growth factor beta 1) in each gram of purified platelet-derived exosome dry powder is 50 ng-20,000 ng; or (5) a content of EGF (epidermal growth factor) in each gram of purified platelet-derived exosome dry powder is 0.1 ng-200 ng.

In some embodiments, in each gram of purified platelet-derived exosome dry powder, a content of PDGF-BB is 0.01, 0.1, 1, 10, 20, 30, 40, 50, 60, 70, 80, 90, 1×10², 5×10², 1×10³, 1.5×10³, or 2×10⁴ ng.

In some embodiments, in each gram of purified platelet-derived exosome dry powder, a content of VEGF is 50, 100, 150, 200, 250, 300, 350, 400, 450, 500, 550, 600, 650, 700, 750, 800, 850, 900, 950, 1×10³, 2×10³, 3×10³, 4x10³, 5×10³, 6×10³, 7×10³, 8×10³, 9×10³, 1×10⁴, 1.5×10⁴, 2×10⁴, 2.5×10⁴, 3×10⁴, 3.5×10⁴, 4×10⁴, 4.5×10⁴, 5×10⁴, 6×10⁴, 7×10⁴, 8×10⁴, 9×10⁴, or 1×10⁵ pg.

In some embodiments, in each gram of purified platelet-derived exosome dry powder, a content of IGF is 1, 10, 20, 30, 40, 50, 60, 70, 80, 90, 1×10², 5×10², 1×10³, 1.5× 10³, 2×10³, 2.5×10³, or 3×10³ pg.

In some embodiments, in each gram of purified platelet-derived exosome dry powder, a content of TGF-B1 is 50, 100, 200, 300, 400, 500, 600, 700, 800, 900, 1×10³, 5×10³, 1×10⁴, 1.5×10⁴, or 2×10⁴ ng.

In some embodiments, in each gram of growth factor enriched dry powder, a content of EGF is 1, 10, 20, 30, 40, 50, 60, 70, 80, 90, 100, 150, or 200 ng.

In some embodiments, (1) no white blood cell-derived exosome is detectable in each gram of purified platelet-derived exosome dry powder, or (2) a number of white blood cell-derived exosomes in each gram of purified platelet-derived exosome dry powder is less than or equal to 1×10¹⁴; wherein these white blood cell-derived exosomes contain a white blood cell marker, which is CD45.

In some embodiments, (1) no red blood cell-derived exosome is detectable in each gram of purified platelet-derived exosome dry powder, or (2) a number of red blood cell-derived exosomes in each gram of purified platelet-derived exosome dry powder is less than or equal to 1 ×10¹⁴; wherein these red blood cell-derived exosomes contain a red blood cell marker, which is at least one of CD235a or Annexin V, or a combination thereof.

In some embodiments, (1) no exosome other than the platelet-derived exosome is detectable in each gram of purified platelet-derived exosome dry powder; or (2) the number of non-platelet-derived exosomes in each gram of the purified platelet-derived exosome dry powder is less than or equal to 1×10¹⁴.

The present invention further provides a use of purified platelet-derived exosome dry powder in the manufacture of a pharmaceutical or health composition for administering an effective dose to a site of an individual to relieve a degree of inflammation or injury at that site of the individual.

In some embodiments, the purified platelet-derived exosome dry power is used in manufacture of the pharmaceutical composition or the health composition to relieve the degree of inflammation or injury in a respiratory tract region, a skin region, a muscle region, a tendon region, a bone region, a joint region, or a ligament region of the individual.

The present invention further provides a use of purified platelet-derived exosome dry powder in the manufacture of a pharmaceutical or health composition to enhance a migration capability of human dermal fibroblasts into a skin region.

In some embodiments, the skin region is a skin lesion site.

This invention further provides a use of purified platelet-derived exosome dry powder in the manufacture of a pharmaceutical or health composition to enhance a collagen expression level of human dermal fibroblasts.

### [BRIEF DESCRIPTION OF THE DRAWING]

Figure 1: Flowchart of preparing purified platelet-derived exosome dry powder.
Figure 2: The bar chart of the effects of different activation methods on the release level of platelet-derived exosomes and PDGF-BB.
Figure 3: The bar chart of the effects of different activation methods on the concentration of platelet-derived exosomes.
Figure 4: The bar chart of the effect of freeze-thaw cycles on the concentration of platelet-derived exosomes.
Figure 5A: The bar chart of the effect of freeze-thawing on the concentration of platelet-derived exosomes.
Figure 5B: The bar chart of the effect of freeze-thawing on total protein amount.
Figure 6A: The bar chart of the effect of platelet-derived exosome compositions obtained from freeze-thaw activation procedures on the cell migration of human dermal fibroblasts.
Figure 6B: The bar chart of the effect of platelet-derived exosome compositions obtained from freeze-thaw activation procedures on the cell proliferation of human dermal fibroblasts.
Figure 7: The bar chart of analyzing the exosome composition in different biological solutions (platelets and plasma).
Figure 8: The bar chart of the effects of exosomes in different biological solutions (platelets and plasma) on alleviating inflammation in human dermal fibroblasts.
Figure 9: The bar chart of the effect of the freeze-drying process on the anti-inflammatory efficacy of the product.
Figure 10A: The bar chart of the effects of platelet-derived exosomes and mesenchymal stromal/stem cell-derived exosomes on the cell migration of human dermal fibroblasts.
Figure 10B: The bar chart of the effects of platelet-derived exosomes and mesenchymal stromal/stem cell-derived exosomes on the cell proliferation of human dermal fibroblasts.
Figure 10C: The bar chart of the effects of platelet-derived exosomes and mesenchymal stromal/stem cell-derived exosomes on collagen expression in human dermal fibroblasts.

### [DETAILED DESCRIPTION OF THE INVENTION]

The following is a detailed description of the embodiments of the present invention as well as the technology and features of the present invention. However, these embodiments are not intended to limit the present invention. Any changes and modifications made by anyone familiar with the technology without departing from the spirit and scope of the present invention should be included in the scope of the patent application of the present invention

In the present invention, the "purified platelet-derived exosome dry powder" refers to a dry powder enriched in high-purity platelet-derived exosomes. For example, each gram of dry powder contains at least 1×10¹⁰ exosomes, in which 80%, 85%, 90%, 95%, 96%, 97%, 98%, 99%, or more than 99.5% of these exosomes are platelet-derived exosomes.

In the present invention, the "purified platelet-derived exosome" dry powder contains fewer other exosomes compared with the source composition from which the dry powder is derived, in which "other exosomes" refer to exosomes other than platelet-derived exosomes.

In the present invention, the purified platelet-derived exosome solution refers to a solution enriched in high-purity platelet-derived exosomes (pure platelet-derived exosomes).

In the present invention, the "purified platelet-derived exosome" solution contains fewer other exosomes compared with the source composition from which the solution is derived, in which "other exosomes" refer to exosomes other than platelet-derived exosomes.

In the present invention, "one unit" platelet-containing solution refers to a capacity of a container of any size for a platelet-containing solution. For example, a blood bag has a capacity of 250 mL for a platelet-containing solution, a 50-mL centrifuge tube has a capacity of 50 mL for a platelet-containing solution, and a 1-mL container has a capacity of 1 mL for a platelet-containing solution.

In the present invention, the "pure platelet solution" refers to a higher-purity platelet solution purified from a platelet-containing solution.

In the present invention, the "platelet-associated structures" refer to platelets, platelet fragments, or platelet structures formed by platelet aggregation, or any combination thereof.

In the present invention, the "sedimentation process" refers to a procedure that causes substances to settle and accumulate such as the procedure of using gravitational force, centrifugal force, or electromagnetic force to make substances settle and accumulate.

In the present invention, "health composition" or "health product" refers to a nutritional supplement, a composition that can promote the maintenance of normal physiological functions in an individual's tissues when administered to an individual, or a composition that can alleviate an individual's symptoms when administered to an individual.

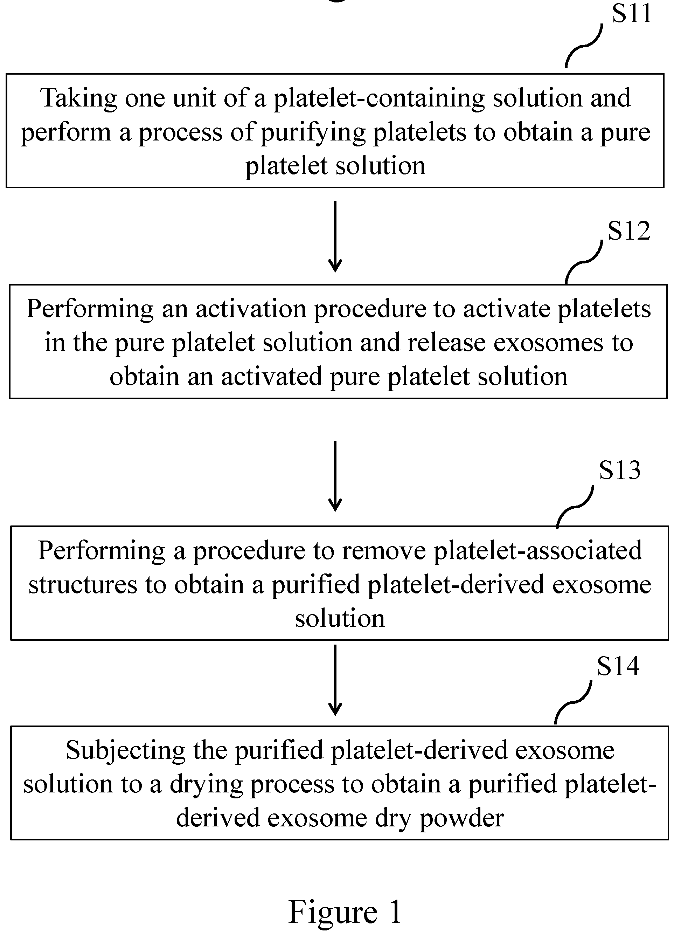

Please refer to Figure 1. The preparation of purified platelet-derived exosome dry powder comprises the following steps:
(S11) taking one unit of platelet-containing solution and perform a process of purifying platelets to obtain a pure platelet solution;
(S12) performing an activation procedure to activate platelets in the pure platelet solution and release exosomes to obtain an activated pure platelet solution;
(S13) performing a procedure to remove platelet-associated structures to obtain a purified platelet-derived exosome solution;
(S14) subjecting the purified platelet-derived exosome solution to a drying process to obtain a purified platelet-derived exosome dry powder containing a plurality of platelet-derived exosomes.

In some embodiments, the platelet-containing solution is derived from blood. In some embodiments, the platelet-containing solution is whole blood, apheresis platelets, leukocyte-reduced apheresis platelets, or platelet-rich plasma (PRP), or any combination thereof. In some embodiments, the platelet-containing solution is derived from an autologous blood sample or an allogeneic blood sample, or a combination thereof.

In some embodiments, step (S11) further comprises the following sub-steps:
(S111) Taking one unit of a solution derived from blood and containing platelets;
(S112) Performing at least one sedimentation process to separate the solution derived from blood and containing platelets into multiple layers, in which the multiple layers comprise a platelet layer (e.g., the first platelet layer) and a white blood cell layer. Removing the white blood cell layer to obtain the pure platelet solution, ensuring that the number of white blood cells in each milliliter (mL) of the pure platelet solution is less than 10⁶. Alternatively, performing at least one sedimentation process to separate the solution containing platelets into multiple layers, in which the multiple layers comprise a platelet layer (e.g., the second platelet layer) and a plasma and plasma protein layer. Removing the plasma and plasma protein layer to obtain the pure platelet solution, ensuring that the albumin concentration in the pure platelet solution is less than 3 g/dL, or the globulin concentration in the pure platelet solution is less than 2 g/dL, or the fibrinogen concentration in the pure platelet solution is less than 100 µg/mL.

In some embodiments, step (S112) further comprises the following sub-steps:
(S1121) Performing a first sedimentation process to separate the platelet-containing solution into a first platelet layer (e.g., plasma layer), a white blood cell layer (e.g., a buffy coat layer), and a red blood cell layer;
(S 1122) Removing the buffy coat layer and red blood cell layer to obtain a plasma layer solution;
(S 1123) Performing a second sedimentation process to settle the platelets into a pellet. The pellet is the second platelet layer. The supernatant is a plasma and plasma protein layer;
(S 1124) Removing the supernatant and mix the pellet with a solvent to obtain a pure platelet solution.

In some embodiments, the solvent is sterile saline, water for injection, 0.45% NaCl, 4.5% hypertonic saline, sterile warm spring water, or isotonic saline, or any combination thereof. In some embodiments, the platelet concentration in the pure platelet solution is 1 ×10⁹/mL-1 0× 10⁹/mL.

In some embodiments, the activation procedure in step (S12) is a freezing and then thawing procedure (i.e., freeze-thaw procedure), and the step (S12) further comprises the following sub-steps:
(S121) Placing a container containing the pure platelet solution at -70°C to - 196°C to freeze the pure platelet solution;
(S122) Raising the temperature of the container to thaw the frozen pure platelet solution, thereby activating the platelets in the pure platelet solution and causing them to release exosomes.
(S123) Performing steps (S121) and (S122) 1, 2, 3, 4, or 5 times to obtain the activated pure platelet solution.

In some embodiments, step (S13) further comprises the following sub-steps:
(S131) Performing a centrifugation process to remove most platelet-associated structures.
(S132) Performing a filter membrane filtration process to completely remove the platelet-associated structures, thereby obtaining the purified platelet-derived exosome solution.

In some embodiments, the drying process in step (S14) is a freeze-drying procedure. In some embodiments, the freeze-drying procedure in step (S14) comprises lowering the temperature to less than or equal to -30°C and lowering the pressure to less than or equal to 100 mTorr. In some embodiments, the freeze-drying procedure in step (S14) comprises lowering the temperature to less than or equal to -35°C and lowering the pressure to less than or equal to 80 mTorr.

Experiment 1: Preparation of purified platelet-derived exosome dry powder and analysis of the composition thereof.

In this experiment, purified platelet-derived exosome dry powder was prepared through the following steps (S11')-(S14'):
(S11') The procedure for preparing the pure platelet solution was performed, which included the following sub-steps:
(S111') 250 mL of human whole blood was placed into an anticoagulant-containing blood bag;
(S112') The process of purifying platelets was executed, which included the following sub-steps:
   (S1121') The human whole blood was centrifuged using a centrifuge (500-1,200g, 5-8 minutes; this was the first centrifugation) to separate the human whole blood into three layers, from top to bottom: the plasma layer, buffy coat layer (where white blood cells reside), and red blood cell layer. The stratification of blood cells after centrifugation is well known to those skilled in the art, and the centrifugation conditions for the first centrifugation can be expanded to 300-1,500g for 3-10 minutes. Platelets were distributed in the plasma layer and were located near the buffy coat layer after centrifugation.
   (S1122') The plasma layer was aseptically aspirated to completely remove white blood cells and red blood cells. The resulting solution is the platelet-rich plasma (PRP) in the present invention, which is also referred to as the plasma layer solution in this application;
   (S1123') The total platelet count in the plasma layer solution was measured using an automated hematology analyzer (model: XP-300, brand: SYSMEX). The plasma layer solution was then centrifuged using a centrifuge (1,000-2,500g, 5 minutes; this was the second centrifugation) to settle the platelets into a pellet. The pellet was the platelet layer, and the supernatant was the plasma and plasma protein layer. The centrifugation conditions for the second centrifugation can be expanded to 500-3,000g for 5-20 minutes, and the centrifugation speed must exceed the actual centrifugation speed used in the aforementioned first centrifugation step (300-1,500g, 3-10 minutes);
   (S1124') The supernatant was removed entirely to eliminate plasma and plasma proteins. Platelets in the platelet layer were then suspended in isotonic sodium chloride solution injection (making a platelet concentration 1×10⁹/mL, with a total volume of 10-20 mL) to obtain a pure platelet solution.
   (S12') An activation procedure was executed, which included the following sub-steps:
      (S121') A cryovial containing the pure platelet solution was placed at -80°C and allowed to sit for 24-36 hours (the freezing time can be extended to 12-60 hours) to freeze the pure platelet solution. Among these, the cryovial contained 1×10⁹ platelets, and the platelet concentration in the pure platelet solution was 1×10⁹ per mL.
      (S 122') The cryovial was placed in a 37°C water bath to thaw the frozen pure platelet solution, thereby activating the platelets in the pure platelet solution, causing them to release exosomes.
      (S123') Steps (S121') and (S122') were repeated twice (i.e., freezing and thawing a total of three times) to obtain the activated pure platelet solution.
      (S13') A purification process was conducted to remove platelet-associated structures, which included the following sub-steps:
         (S131') The activated pure platelet solution was centrifuged using a centrifuge (10,000-15,000g, 5-10 minutes) to settle the platelet-associated structures into a platelet-associated structure pellet. The centrifugation conditions can be expanded to 8,000-20,000g, 3-15 minutes.
         (S 132') The supernatant was carefully transferred into a specimen bottle and filtered through 0.45 µm pore size filter membranes (material: PES, 25 mm, model: C0000296, brand: Labfil^{®}) to remove the platelet-associated structure pellet completely. The resulting supernatant was the purified platelet-derived exosome solution. Among these, the specimen bottle contained approximately 1 mL of purified platelet-derived exosome solution, and the approximately 1 mL of purified platelet-derived exosome solution comprised platelet-derived exosomes released from 1×10⁹ platelets. Nanoparticle quantitative analysis was then performed with a nanoparticle size analyzer (Nano Tracking Analysis, NTA, model: NanoSight NS300, brand: Malvern). The analysis results indicated that the exosome content in the approximately 1 ml of purified platelet-derived exosome solution was about 1 ×10⁹-1×10¹², with particle size ranging from 20-150 nm. Subsequently, a fully automated exosome fluorescence quantitative analyzer (ExoView, brand: Leprechaun) was used to determine whether these exosomes contained platelet-derived exosome biomarkers. It was found that these exosomes contained exosome markers CD9, CD63, and CD81, as well as platelet markers CD41 and CD42b. Therefore, these exosomes were indeed platelet-derived exosomes. Furthermore, these exosomes lacked the white blood cell marker CD45 and the red blood cell marker CD235a or Annexin V. It was concluded that the purified platelet-derived exosome dry powder prepared in Experiment 1, steps (S11')-(S14'), of this application did not contain white blood cell-derived exosomes or red blood cell-derived exosomes.
         (S14') A freeze-drying procedure was performed that included the following sub-steps:
            (S141') Pre-cooling (-30 to -50°C, at least 5 hours);
            (S142') Primary drying (the temperature gradually increased from the temperature in step S141' to 10°C over 10-20 hours under a vacuum level of less than or equal to 100 mTorr);
            (S143') Secondary drying (20°C, 3-5 hours with a vacuum level of less than or equal to 100 mTorr). The dried material obtained after freeze-drying the purified platelet-derived exosome solution (approximately 1 mL) in a specimen bottle was one bottle of purified platelet-derived exosome dry powder. The weight of one bottle of purified platelet-derived exosome dry powder was measured using an analytical balance (model: ME204, brand: Mettler). The results showed that the average weight of each bottle of purified platelet-derived exosome dry powder was 0.0067 grams(g). After calculations, it was determined that each gram of purified platelet-derived exosome dry powder contained at least 1×10¹³-10×10¹³ platelet-derived exosomes.

From the above preparation procedure, it could be seen that the process for preparing purified platelet-derived exosome dry powder included the steps for removing white blood cells (WBC) and plasma, activating platelets (e.g., through freezing and thawing), removing platelet-associated structures, and drying (e.g., via a freeze-drying step, a cryogenic drying step, or a vacuum drying step). As a result, the purified platelet-derived exosome dry powder contained a low concentration of, or even no, white blood cells (WBCs), plasma, and platelets.

In some embodiments, the purified platelet-derived exosome dry powder comprised a low concentration of or even no albumin.

In some embodiments, the purified platelet-derived exosome dry powder comprised a low concentration of or even no globulin.

In some embodiments, the purified platelet-derived exosome dry powder comprised a low concentration of or even no fibrinogen.

Experiment 2: Effects of freeze-thawing (activation procedure) on the release levels of platelet-derived exosomes and PDGF-BB.

Step (S11') of Experiment 1 was performed to prepare a pure platelet solution. The platelet concentration in the pure platelet solution was 1×10⁹/mL. Then, the pure platelet solution was divided into three groups, i.e., the control group, the -196°C group, and the freeze-thaw 3-cycle group.

Control group: 1 mL of the pure platelet solution was taken, but the activation procedure of step (S12') in Experiment 1 was not performed. In other words, the pure platelet solution was not placed at -80°C for three freezing and then thawing cycles but was allowed to sit for 20-30 minutes. Subsequently, step (S13') of Experiment 1 was performed to prepare the control group solution.

-196°C group: 1 mL of the pure platelet solution was taken, but the activation procedure of step (S12') in Experiment 1 was not performed. In other words, the pure platelet solution was not placed at -80°C for three freezing and then thawing cycles; instead, it was placed at -196°C to freeze for 20-30 minutes and then thawed at 37°C once. Subsequently, step (S13') of Experiment 1 was performed to prepare the -196°C group solution.

Freeze-thaw 3-cycle group: 1 mL of the pure platelet solution was taken, and steps (S12')-(S13') of Experiment 1 were performed to prepare the freeze-thaw 3-cycle group solution (i.e., the purified platelet-derived exosome solution in this application).

The concentration of platelet-derived exosomes in each group's solution was analyzed using a nanoparticle size analyzer (Tunable Resistive Pulse Sensing (TRPS), model: Exoid, brand: IZON Science). Furthermore, the concentration of growth factors (PDGF-BB) in each group's solution was analyzed using an enzyme-linked immunosorbent assay (ELISA). Then the following formulas were used to calculate platelet-derived exosome release fold and growth factor release fold from each group.Platelet-derived exosome release fold = (platelet-derived exosome concentration in the experimental group solution) ÷ (platelet-derived exosome concentration in the control group solution). Growth factor release fold = (growth factor concentration in the experimental group solution) ÷ (growth factor concentration in the control group solution).

Among these, the experimental group solution was the -196°C or freeze-thaw 3-cycle group solution.

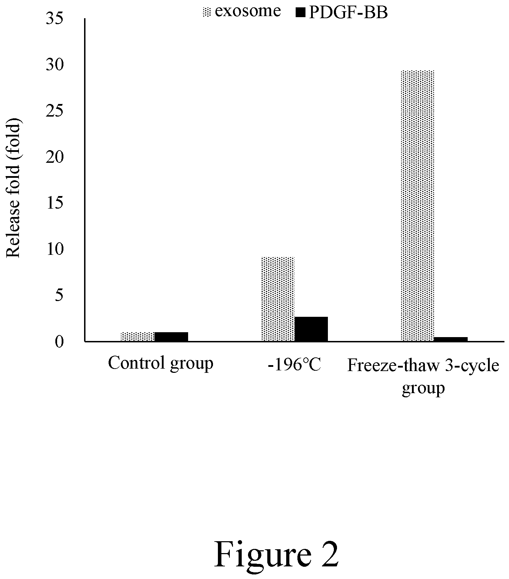

Please refer to Table 1 and Figure 2 for experimental results. Figure 2 is the bar chart of the effects of different activation methods on the release level of platelet-derived exosomes and PDGF-BB.

**Table 1**

| Group | Platelet-derived exosome concentration (particles/ml) | Platelet-derived exosome release fold (fold) | PDGF-BB content (ng/mL) | PDGF-BB release fold (fold) |
|---|---|---|---|---|
| Control group | 9,610,000,000 | 1 | 1.7 | 1 |
| -196°C group | 87,700,000,000 | 9 | 4.5 | 2.65 |
| Freeze-thaw 3-cycle group | 282,000,000,000 | 29 | 0.8 | 0.47 |

The results in Table 1 and Figure 2 show that in the -196°C group, the platelet-derived exosomes release fold was 9 while the PDGF-BB release factor was 2.65. In the freeze-thaw 3-cycle group, the platelet-derived exosomes release fold increased to 29, whereas the PDGF-BB release fold decreased to 0.47. It could be seen that the changing trend of platelet-derived exosomes and the changing trend of PDGF-BB release fold were independent of each other in solutions prepared using different activation methods. Therefore, the inventor further conducted the following experiments to identify the best method for preparing platelet-derived exosomes.

### Experiment 3: The effect of different activation methods on the concentration of exosomes

Step (S11') of Experiment 1 was performed to prepare a pure platelet solution. The platelet concentration in the pure platelet solution was 1×10⁹/mL. Then, the pure platelet solution was divided into a control group and six experimental groups: calcium chloride group, -196°C group, adenosine diphosphate group, collagen group, thrombin group, and freeze-thaw 3-cycle group.

Control group: 1 mL of the pure platelet solution was taken, but the activation procedure of step (S12') in Experiment 1 was not performed. Instead, the pure platelet solution was allowed to sit for 20-30 minutes. Subsequently, the step (S13') of Experiment 1 was performed to prepare the control group solution.

Calcium chloride (CaCl₂) group: 1 mL of the pure platelet solution was taken, but the activation procedure of step (S12') in Experiment 1 was not performed. In other words, the pure platelet solution was not placed at -80°C for three freezing and then thawing cycles. Instead, it was mixed with calcium chloride to achieve the mixture's calcium chloride concentration of 0.45%. The mixture was then placed on a orbital shaker (model: DSR-D-N1, brand: DIGISYSTEM) and allowed to react at 100 rpm at room temperature for 20-30 minutes. Subsequently, step (S13') of Experiment 1 was performed to prepare the calcium chloride group solution.

-196°C group: 1 mL of the pure platelet solution was taken, but the activation procedure of step (S12') in Experiment 1 was not performed. In other words, the pure platelet solution was not placed at -80°C for three freezing and then thawing cycles; instead, it was placed at -196°C to freeze for 20-30 minutes and then thawed at 37°C once. Subsequently, step (S13') of Experiment 1 was performed to prepare the -196°C group solution.

Adenosine diphosphate (ADP) group: 1 mL of the pure platelet solution was taken, but the activation procedure of step (S12') in Experiment 1 was not performed. In other words, the pure platelet solution was not placed at -80°C for three freezing and then thawing cycles. Instead, it was mixed with adenosine diphosphate (ADP, catalog number: A2754, brand: Sigma-Aldrich) to achieve the mixture's ADP concentration of 60 µM. The mixture was then placed on a orbital shaker (model: DSR-D-N1, brand: DIGISYSTEM) and allowed to react at 100 rpm at room temperature for 20-30 minutes. Subsequently, step (S13') of Experiment 1 was performed to prepare the ADP group solution.

Collagen group: 1 mL of the pure platelet solution was taken, but the activation procedure of step (S12') in Experiment 1 was not performed. In other words, the pure platelet solution was not placed at -80°C for three freezing and then thawing cycles. Instead, it was mixed with collagen (C7661, brand: Sigma-Aldrich) to achieve the mixture's collagen concentration of 10 µg/mL. The mixture was then placed on a orbital shaker (model: DSR-D-N1, brand: DIGISYSTEM) and allowed to react at 100 rpm at room temperature for 20-30 minutes. Subsequently, step (S13') of Experiment 1 was performed to prepare the collagen group solution.

Thrombin group: 1 mL of the pure platelet solution was taken, but the activation procedure of step (S12') in Experiment 1 was not performed. In other words, the pure platelet solution was not placed at -80°C for three freezing and then thawing cycles. Instead, it was mixed with thrombin (catalog number: T7326, brand: Sigma-Aldrich) to achieve the mixture's thrombin concentration of 1 U/mL. The mixture was then placed on a rotator and allowed to react at 100 rpm at room temperature for 20-30 minutes. Subsequently, step (S13') of Experiment 1 was performed to prepare the thrombin group solution.

Freeze-thaw 3-cycle group: 1 mL of the pure platelet solution was taken, and steps (S12')-(S13') of Experiment 1 were performed to prepare the freeze-thaw 3-cycle group solution (i.e., the purified platelet-derived exosome solution in this application).

Nanoparticle quantification analysis was performed using a nanoparticle size analyzer (Tunable Resistive Pulse Sensing (TRPS), model: Exoid, brand: IZON Science) to determine the concentration of platelet-derived exosome in each group's solution. Then the following formulas were used to calculate each group's platelet-derived exosome release fold. Experimental group platelet-derived exosome release fold = (platelet-derived exosome concentration in the experimental group solution) ÷ (platelet-derived exosome concentration in the control group solution).

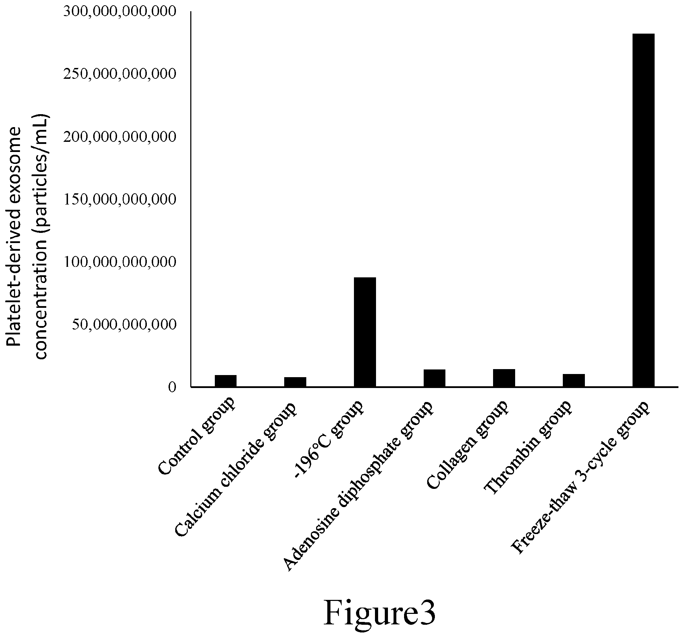

The experimental results are shown in Table 2 and Figure 3.

**Table 2**

| Group | Platelet-derived exosome concentration (particles/ml) | Platelet-derived exosome release fold (fold) |
|---|---|---|
| Control group | 9,610,000,000 | 1 |
| Calcium chloride (CaCl₂) group | 7,850,000,000 | 0.82 |
| -196°C group | 87,700,000,000 | 9.13 |
| Adenosine diphosphate (ADP) group | 14,200,000,000 | 1.48 |
| Collagen group | 14,500,000,000 | 1.51 |
| Thrombin group | 10,300,000,000 | 1.07 |
| Freeze-thaw 3-cycle group | 282,000,000,000 | 29.34 |

Please refer to Figure 3. Figure 3 is the bar chart of the effects of different activation methods on the concentration of platelet-derived exsosomes.

The results in Table 2 and Figure 3 demonstrate that, compared to the control group, activation methods such as calcium chloride, -196°C, ADP, collagen, and thrombin failed to significantly increase the concentration of platelet-derived exosomes. Unexpectedly, using the freeze-thaw 3-cycle activation method resulted in a platelet-derived exosome release fold as high as 29 times. In other words, the activation procedure involving three cycles of freezing at -80°C followed by thawing could substantially enhance the release of platelet-derived exosomes, producing an unanticipated effect.

### Experiment 4: Effects of freeze-thaw cycles on the release level of platelet-derived exosomes

Step (S11') of Experiment 1 was performed to prepare a pure platelet solution. Platelet concentration in the pure platelet solution was 1×10⁹/mL. Then, the pure platelet solution was divided into six experimental groups: 1-cycle group, 1-cycle (-196°C) group, 3-cycle group, 3-cycle (-196°C) group, 5-cycle group, and 5-cycle (-196°C) group.

1-cycle group: 1 mL of the pure platelet solution was taken, and steps (S121')-(S122') of Experiment 1 were performed. However, the activation procedure of step (S123') in Experiment 1 was not performed. In other words, the pure platelet solution was placed at -80°C for freezing and then thawing but only for a single freeze-thaw cycle. Subsequently, step (S13') of Experiment 1 was performed to produce the 1-cycle group solution.

1-cycle (-196°C) group: 1 mL of the pure platelet solution was taken, but the activation procedure of step (S12') in Experiment 1 was not performed. In other words, the pure platelet solution was not placed at -80°C for three freezing and then thawing cycles; instead, it was placed at -196°C to freeze for 20-30 minutes and then thawed at 37°C once. Subsequently, step (S13') of Experiment 1 was performed to prepare the 1-cycle (-196°C) group solution.

3-cycle group: 1 mL of the pure platelet solution was taken, and steps (S12')-(S13') of Experiment 1 were performed to prepare the freeze-thaw 3-cycle group solution (i.e., the purified platelet-derived exosome solution in this application).

3-cycle (-196°C) group: 1 mL of the pure platelet solution was taken, but the activation procedure of step (S12') in Experiment 1 was not performed. In other words, the pure platelet solution was not placed at -80°C for three freezing and then thawing cycles; instead, it was placed at -196°C to freeze for 20-30 minutes and then thawed at 37°C for three cycles. Subsequently, step (S13') of Experiment 1 was performed to prepare the 3-cycle (-196°C) group solution.

5-cycle group: 1 mL of the pure platelet solution was taken, and steps (S121')-(S122') of Experiment 1 were performed. However, the activation procedure of step (S123') in Experiment 1 was not carried out. In other words, although the pure platelet solution was placed at -80°C for freezing and then thawing, it underwent five freeze-thaw cycles instead of three. Subsequently, step (S13') of Experiment 1 was performed to prepare the 5-cycle group solution.

5-cycle (-196°C) group: 1 mL of the pure platelet solution was taken, but the activation procedure of step (S12') in Experiment 1 was not performed. In other words, the pure platelet solution was not placed at -80°C for three freezing and then thawing cycles; instead, it was placed at -196°C to freeze for 20-30 minutes and then thawed at 37°C for five cycles. Subsequently, step (S13') of Experiment 1 was performed to prepare the 5-cycle (-196°C) group solution.

The concentration of platelet-derived exosomes in each group's solution was analyzed using a nanoparticle size analyzer (Tunable Resistive Pulse Sensing (TRPS), model: Exoid, brand: IZON Science).

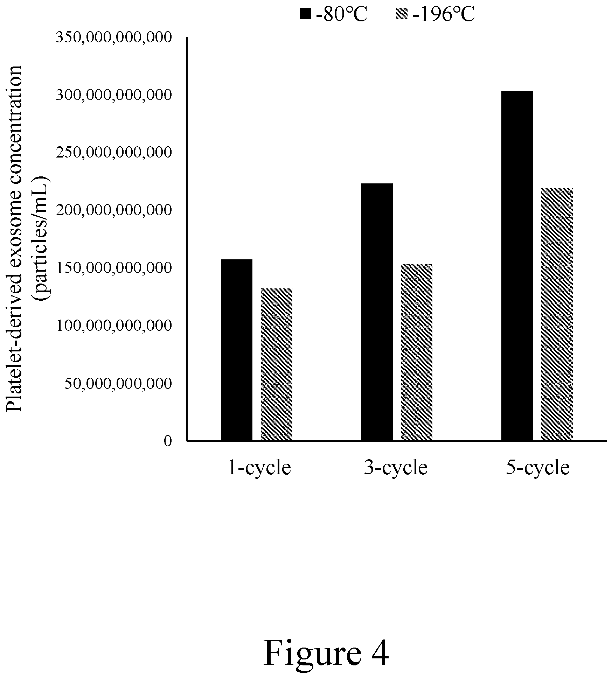

The experimental results are shown in Table 3 and Figure 4.

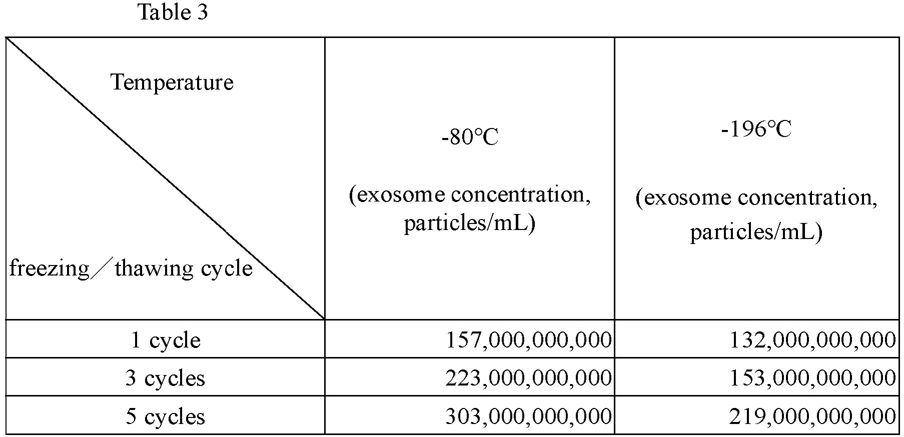

Please refer to Figure 4. Figure 4 is the bar chart of the effect of freeze-thaw cycles on the concentration of platelet-derived exosomes.

The results in Table 3 and Figure 4 show that the platelet-derived exosome concentration in the 1-cycle group solution, 3-cycle group solution, and 5-cycle group solution were all higher than the platelet-derived exosome concentration in the 1-cycle (-196°C) group solution, 3-cycle (-196°C) group solution, and 5-cycle (-196°C) group solution. Furthermore, the platelet-derived exosome concentration in the 3-cycle group solution increased by more than 45% compared with the 3-cycle (-196°C) group solution. These results indicated that regardless of the number of freeze-thaw cycles, the platelet-derived exosome concentration obtained from freeze-thawing at -80°C was consistently higher than the platelet-derived exosome concentration obtained through freeze-thawing at -196°C, showing an unexpected effect. In other words, the activation procedure of freezing at -80°C followed by thawing could achieve the highest release level of platelet-derived exosomes.

Experiment 5: The effect of freeze-thaw cycles on the release level of platelet-derived exosomes and total protein.

Step (S11') of Experiment 1 was performed to prepare a pure platelet solution. The platelet concentration in the pure platelet solution was 1×10⁹/mL. Then, the pure platelet solution was divided into a control and freeze-thaw 3-cycle group.

Control group: 1 mL of the pure platelet solution was taken, but the activation procedure of step (S12') in Experiment 1 was not performed. Instead, the pure platelet solution was allowed to sit for 20-30 minutes. Then, step (S13') of Experiment 1 was performed to prepare the control group solution.

Freeze-thaw 3-cycle group: 1 mL of the pure platelet solution was taken, and steps (S12')-(S13') of Experiment 1 were performed to prepare the freeze-thaw 3-cycle group solution (i.e., the purified platelet-derived exosome solution in this application).

The concentration of platelet-derived exosomes in the two group's solution was analyzed using a nanoparticle size analyzer (Tunable Resistive Pulse Sensing (TRPS), model: Exoid, brand: IZON Science). Additionally, total protein quantification in each solution was performed using an automatic biochemical analyzer (TOSHIBA TBA-120FR).

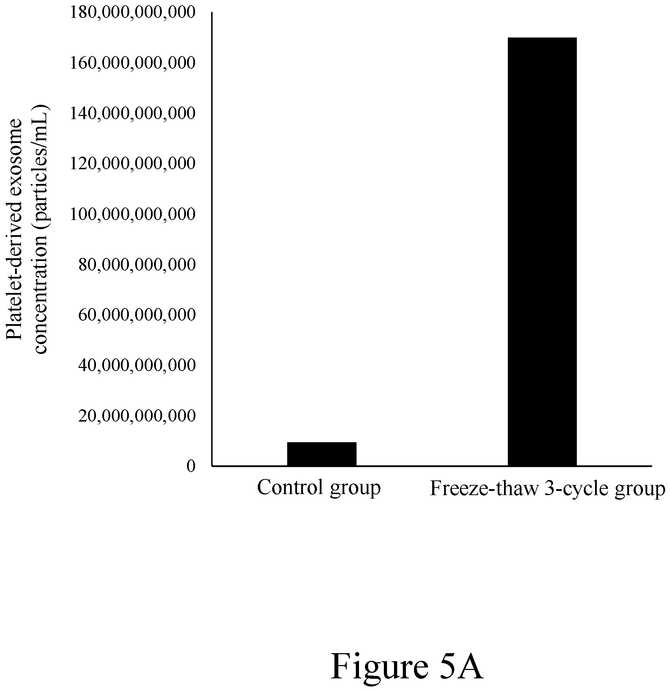

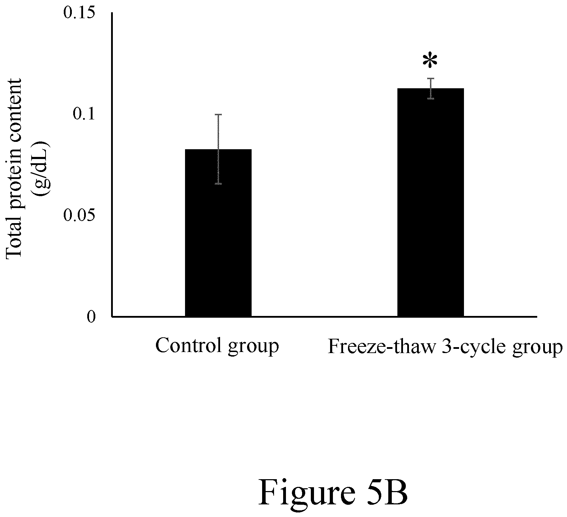

The experimental results for platelet-derived exosome concentration are shown in Table 4 and Figure 5A. The experimental results for total protein content are shown in Table 4 and Figure 5B. Data were presented as mean ± SD, and statistical analysis was performed using a t-test. Groups marked with "*" indicated a statistically significant difference compared with the control group (p < 0.05).

**Table 4**

| Group | Platelet-derived exosome concentration (particles/mL) | Average total protein content (g/dL) |
|---|---|---|
| Control group | 9,450,000,000 | 0.0825 |
| Freeze-thaw 3-cycle group | 170,000,000,000 | 0.1125 |

Please refer to Figures 5A and 5B. Figure 5A is the bar chart of the effect of freeze-thawing on the concentration of platelet-derived exosomes. Figure 5B is the bar chart of the effect of freeze-thawing on total protein amount.

The results in Table 4 and Figure 5A show that the platelet-derived exosome concentration in the control group was 9,450,000,000 particles/mL; the platelet-derived exosome concentration in the freeze-thaw 3-cycle group was 170,000,000,000 particles/mL. It could be seen that the platelet-derived exosome concentration in the freeze-thaw 3-cycle group was significantly higher than that in the control group.

The results in Table 4 and Figure 5B showed that the average total protein content in the control group was 0.0852 g/dL while the average total protein content in the freeze-thaw 3-cycle group was 0.1125 g/dL.

Experiment 6: Effects of platelet-derived exosome compositions obtained from freeze-thaw activation procedures on alleviating inflammation and tissue repair.

Experiment 6-1: Effects of platelet-derived exosome compositions obtained from freeze-thaw activation procedures on alleviating inflammation.

Human dermal fibroblasts (HDFs) (purchased from ScienCell Research Laboratories, Inc., catalog number: 2320) were cultured in Dulbecco's Modified Eagle Medium (DMEM, Corning 10-013CV) containing 2% fetal bovine serum (FBS), followed by replacing the culture medium with fresh medium for later use. The HDFs are adherent cells.

Step (S11') of Experiment 1 was performed to prepare a pure platelet solution. The platelet concentration in the pure platelet solution was 1×10⁹/mL. Then, the pure platelet solution was divided into control and freeze-thaw 3-cycle group.

Control group: 1 mL of the pure platelet solution was taken, but the activation procedure of step (S12') in Experiment 1 was not performed. Instead, the pure platelet solution was allowed to sit for 20-30 minutes. Then, step (S13') of Experiment 1 was performed to prepare the control group solution. The control group solution was mixed with DMEM containing 2% FBS (the volume ratio of the control group solution and DMEM containing 2% FBS was 1:19) to prepare the control group's platelet-derived exosome medium.

Freeze-thaw 3-cycle group: 1 mL of the pure platelet solution was taken, and steps (S12')-(S13') of Experiment 1 were performed to prepare the freeze-thaw 3-cycle group solution (i.e., the purified platelet-derived exosome solution in this application). The freeze-thaw 3-cycle group solution was mixed with DMEM containing 2% FBS (the volume ratio of the freeze-thaw 3-cycle group solution and DMEM containing 2% FBS was 1:19) to prepare the three cycles of freezing and thawing group's platelet-derived exosome medium.

A 96-well plate was divided into untreated wells, LPS control group wells, control group exosome solution wells, and freeze-thaw 3-cycle group exosome solution wells, with two replicates per group. HDFs were added to the untreated wells, LPS control group wells, control group exosome solution wells, and freeze-thaw 3-cycle group exosome solution wells, ensuring an equal cell number each well. The cells were incubated for 24 hours in a 37°C, 5% carbon dioxide (CO₂) incubator.

The used medium was removed. Cells in the LPS control group wells, control group exosome solution wells, and freeze-thaw 3-cycle group exosome solution wells were suspended in DMEM containing 5 µg/mL LPS and 2% FBS. Cells in the untreated wells were suspended in DMEM containing 2% FBS without LPS. The cells were incubated for 1 hour in a 37°C, 5% carbon dioxide (CO₂) incubator. Subsequently, the following steps were performed separately for each group.

Untreated wells: The used medium was removed. Cells in the untreated wells control wells) were suspended in DMEM containing 2% FBS. The cells were incubated for an additional 24 hours in a 37°C, 5% carbon dioxide (CO₂) incubator.

LPS control wells: The used medium was removed. Cells in the LPS control wells were suspended in DMEM containing 2% FBS. The cells were incubated for an additional 24 hours in a 37°C, 5% CO₂ incubator.

Control group exosome solution wells: The used medium was removed. Cells in the control group exosome solution wells were suspended in the control group's platelet-derived exosome medium. The cells were incubated for an additional 24 hours in a 37°C, 5% carbon dioxide (CO₂) incubator.

Freeze-thaw 3-cycle group exosome solution wells: The used medium was removed. Cells in the freeze-thaw 3-cycle group exosome solution wells were suspended in the three cycles of freezing and thawing group's platelet-derived exosome medium. The cells were incubated for an additional 24 hours in a 37°C, 5% carbon dioxide (CO₂) incubator.

The cell culture medium from the untreated wells, LPS control wells, control group exosome solution wells, and freeze-thaw 3-cycle group exosome solution wells were transferred into separate microcentrifuge tubes. The concentration of human interleukin-6 (human IL-6, an inflammatory factor) in the cell culture medium was measured using an IL-6 ELISA kit (Human IL-6 ELISA Kit, catalog number: ab178013, brand: Abcam). The analysis was then performed with a multimode microplate spectrophotometer (model: Varioskan LUX, brand: Thermo Scientific) set to an absorbance wavelength of 450 nm. A regression curve of absorbance versus inflammatory factor IL-6 concentration was created based on the instructions of the ELISA kit, and the absorbance values were converted into inflammatory factor IL-6 concentrations.

The data showed that the freeze-thaw 3-cycle group exosome solution was able to inhibit inflammation in HDFs, thereby alleviating inflammation in skin tissue. The inventor expects that after using stimulants (such as LPS) inducing inflammation in fibroblasts from other human tissues, the freeze-thaw 3-cycle group exosome solution can also inhibit the induced inflammatory response, thereby alleviating inflammation in those tissues.

Experiment 6-2: Effects of platelet-derived exosome compositions obtained from freeze-thaw activation procedures on cell migration.

Cell migration and proliferation are beneficial for tissue repair (refer to Reference 1). Human dermal fibroblasts (HDFs) identical to those used in Experiment 6-1 were utilized for this experiment. HDFs were cultured in Dulbecco's Modified Eagle Medium (DMEM, Corning 10-013CV) containing 2% fetal bovine serum (FBS), followed by replacing the culture medium with fresh medium for later use.

A 96-well plate with cell culture inserts was divided into untreated wells, control group exosome solution wells, and freeze-thaw 3-cycle group exosome solution wells, with three replicates per group. HDFs were added to the untreated wells, control group exosome solution wells, and freeze-thaw 3-cycle group exosome solution wells, ensuring an equal cell number each well. The cells were cultured for 24 hours in a 37°C, 5% carbon dioxide (CO₂) incubator. The used medium and each group's cell culture inserts were removed, and the plate was documented by taking photographs under a microscope. It was confirmed that the areas originally occupied by the inserts in each group's wells were not covered by cells. Then, the following steps were performed separately for each group.

Untreated wells: Cells were cultured in DMEM containing 2% FBS for an additional 6 hours in a 37°C, 5% carbon dioxide (CO₂) incubator.

Control group exosome solution wells: Cells in the control group exosome solution wells were suspended in the control group's platelet-derived exosome medium described in Experiment 6-1. The cells were then incubated for an additional 6 hours in a 37°C, 5% carbon dioxide (CO₂) incubator.

Freeze-thaw 3-cycle group exosome solution wells: Cells in the freeze-thaw 3-cycle group exosome solution wells were suspended in the three cycles of freezing and thawing group's platelet-derived exosome medium described in Experiment 6-1. The cells were incubated for an additional 6 hours in a 37°C, 5% carbon dioxide (CO₂) incubator.

Photographs were taken using a microscope to document the results, and the cell migration coverage area of each group was calculated. Data were presented as mean ± SD, and statistical analysis was performed using a t-test. Groups marked with "***" indicated a statistically significant difference compared with the control group exosome solution (p < 0.001).

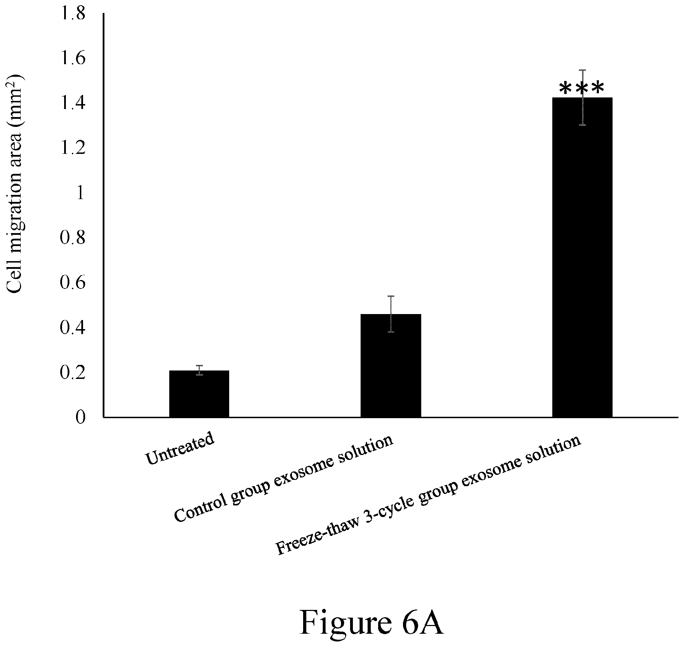

The experimental results are shown in Figure 6A. Figure 6A is the bar chart of the effect of platelet-derived exosome compositions obtained from freeze-thaw activation procedures on the cell migration of human dermal fibroblasts.

The results in Figure 6A show that the average cell migration coverage area was 0.21 mm² in the untreated group; the average cell migration coverage area was 0.46 mm² in the control group exosome solution group; the average cell migration coverage area was 1.42 mm² in the freeze-thaw 3-cycle group exosome solution group. There was a significant difference in cell migration coverage area between the freeze-thaw 3-cycle group exosome solution group and the control group exosome solution group (p < 0.001). This indicated that the platelet-derived exosome composition obtained from the -80°C freeze-thaw activation procedure in this application could promote the migration of HDFs, thereby facilitating tissue repair. Furthermore, the experimental results from the control and freeze-thaw 3-cycle group in Figure 2 demonstrated that the cell migration shown in Figure 6A was induced by the platelet-derived exosomes, not by PDGF-BB.

Experiment 6-3: Effects of platelet-derived exosome compositions obtained from freeze-thaw activation procedures on cell proliferation.

Human dermal fibroblasts (HDFs) identical to those used in Experiment 6-1 were utilized for this experiment. HDFs were cultured in Dulbecco's Modified Eagle Medium (DMEM, Corning 10-013CV) containing 2% fetal bovine serum (FBS), followed by replacing the culture medium with fresh medium for later use.

A 96-well plate was divided into untreated wells, control group exosome solution wells, and freeze-thaw 3-cycle group exosome solution wells, with three replicates per group. HDFs were added to the untreated wells, control group exosome solution wells, and freeze-thaw 3-cycle group exosome solution wells, ensuring an equal cell number each well. The cells were cultured for 24 hours in a 37°C, 5% carbon dioxide (CO₂) incubator. Then, the following steps were performed for each group.

Untreated wells: The used medium was removed. Cells were cultured in DMEM containing 2% FBS for an additional 6 hours in a 37°C, 5% carbon dioxide (CO₂) incubator.

Control group exosome solution wells: The used medium was removed. Cells in the control group exosome solution wells were suspended using the control group's platelet-derived exosome medium described in Experiment 6-1. Cells were incubated for an additional 6 hours in a 37°C, 5% carbon dioxide (CO₂) incubator.

Freeze-thaw 3-cycle group exosome solution wells: Cells in the freeze-thaw 3-cycle group exosome solution wells were suspended using the three cycles freezing and thawing group's platelet-derived exosome medium described in Experiment 6-1. Cells were incubated for an additional 6 hours in a 37°C, 5% carbon dioxide (CO₂) incubator.

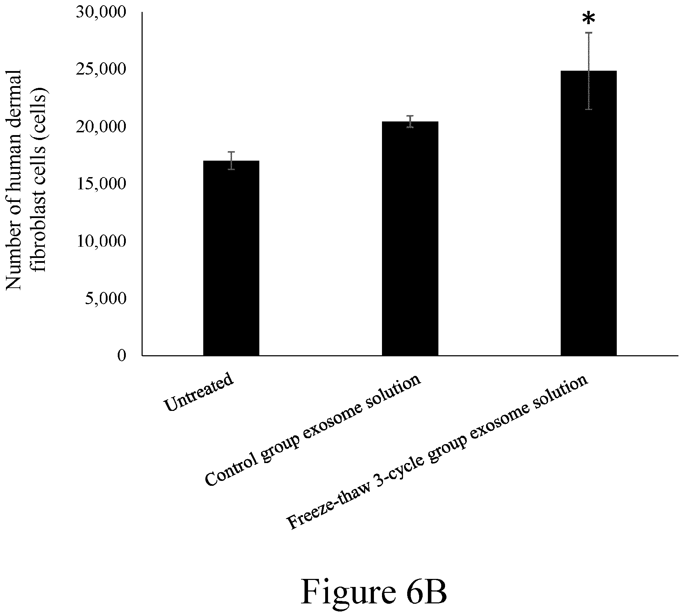

The used medium was removed. 90 µL of DMEM and 10 µL of CCK-8 buffer (DOJINDO, catalog number: CK04) were added to each well. The plate was incubated for an additional 4 hours in a 37°C, 5% carbon dioxide (CO₂) incubator. Analysis was performed with a multimode microplate spectrophotometer (model: Varioskan LUX, brand: Thermo Scientific) set to an absorbance wavelength of 450 nm. A regression curve of absorbance versus cell numbers was created based on the instructions of the CCK-8 buffer, and the absorbance values were converted into viable cell numbers. Data were presented as mean ± SD, and statistical analysis was performed using a t-test. Groups marked with "*" indicated a statistically significant difference compared with the control group exosome solution wells (p < 0.05). The experimental results are shown in Figure 6B. Figure 6B is the bar chart of the effect of platelet-derived exosome compositions obtained from freeze-thaw activation procedures on the cell proliferation of human dermal fibroblasts.

The results in Figure 6B show that the average cell number increased to 17,010 cells/mL in the untreated group; the average cell number increased to 20,425 cells/mL in the control group exosome solution group; the average cell number increased to 24,836 cells/mL in the freeze-thaw 3-cycle group exosome solution group. Among these, there was a significant difference in cell numbers between the freeze-thaw 3-cycle group exosome solution group and the control group exosome solution group (p < 0.05). This demonstrated that the platelet-derived exosome composition obtained from the -80°C freeze-thaw activation procedure in this application could promote the proliferation of HDFs, thereby facilitating tissue repair.

### Experiment 7: Effects of exosomes in different biological solutions on alleviating inflammation

### Experiment 7-1: Analysis of exosome composition in different biological solutions

This experiment was divided into two groups, i.e., the pure platelet and platelet plasma group. Then, the following steps were performed separately for each group.

Pure platelet group: 1 mL of the pure platelet solution described in Experiment 1 was taken, and steps (S12')-(S13') of Experiment 1 were performed to prepare the pure platelet group exosome solution (i.e., the purified platelet-derived exosome solution in this application).

Platelet plasma group: 1 mL of the platelet-rich plasma (PRP) described in Experiment 1 was taken. Then, steps (S12')-(S13') of Experiment 1 were conducted directly to prepare the platelet plasma group exosome solution.

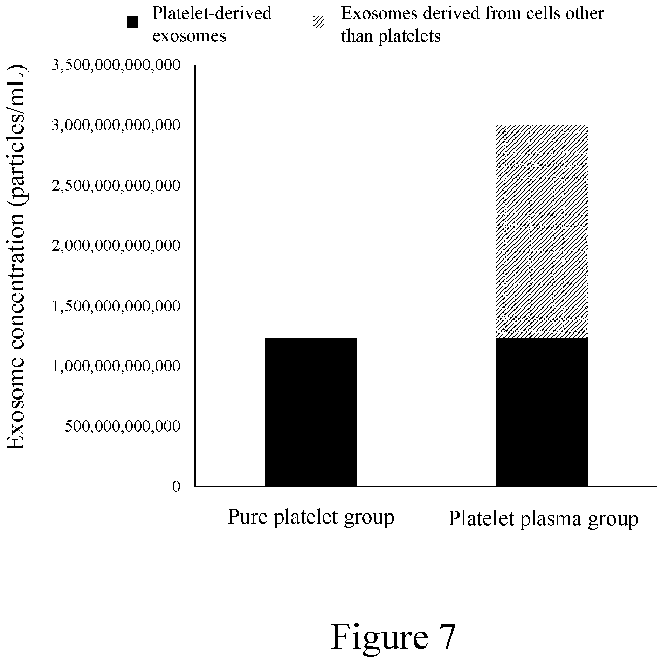

The concentration of exosomes in each group's solution was analyzed using a nanoparticle size analyzer (Tunable Resistive Pulse Sensing (TRPS), model: Exoid, brand: IZON Science). Subsequently, biomarkers of exosomes in the pure platelet group exosome solution and the platelet plasma group exosome solution were individually analyzed using a fully automated exosome fluorescence quantitative analyzer (ExoView, Leprechaun). The experimental results showed that the exosomes in the pure platelet group exosome solution contained exosome markers CD9, CD63, and/or CD81 as well as platelet markers CD41 and CD42b, while they did not possess white blood cell marker CD45 and did not possess at least one of the red blood cell exosome markers CD235a or Annexin V. This indicated that the exosomes in the pure platelet group were 100% platelet-derived. Conversely, the exosomes in the platelet plasma group exosome solution contained at least one of the red blood cell biomarkers CD235a or Annexin V, or the white blood cell biomarker CD45. The experimental results showed that only 41% of the exosomes in the platelet plasma group were platelet-derived, while 59% were exosomes derived from other cell types. Please refer to Table 5 and Figure 7 for the experimental results. Figure 7 is the bar chart of anallyzing the exosome composition in different biological solutions (platelets and plasma).

**Table 5**

| Group | Platelet-derived exosome concentration (particles/mL) | Concentration of exosomes derived from cells other than platelets (particles/mL) |
|---|---|---|
| Pure platelet group | 1,230,000,000,000 | 0 |
| Platelet plasma group | 1,230,000,000,000 | 1,770,000,000,000 |

### Experiment 7-2: Effects of exosomes in different biological solutions on alleviating inflammation

The concentration of the pure platelet group exosome solution prepared in Experiment 7-1 and the platelet plasma group exosome solution prepared in Experiment 7-1 were adjusted using isotonic sodium chloride solution injection, ensuring that both solutions had the same exosome concentration (the total number of exosomes per unit volume was identical). Then, 1 mL of each solution was taken, and the pure platelet group exosome dry powder and platelet plasma group exosome dry powder were prepared according to step (S14') of Experiment 1.

Human dermal fibroblasts (HDFs) identical to those used in Experiment 6-1 were utilized for this experiment. HDFs were cultured in Dulbecco's Modified Eagle Medium (DMEM, Corning 10-013CV) containing 2% fetal bovine serum (FBS), followed by replacing the culture medium with fresh medium for later use.

A 96-well plate was divided into untreated wells, LPS control group wells, platelet plasma experimental group wells, and purified platelet experimental group wells, with two replicates per group. HDFs were added to the untreated wells, LPS control group wells, platelet plasma experimental group wells, and purified platelet experimental group wells, ensuring an equal cell number each well. The cells were cultured for 24 hours in a 37°C, 5% carbon dioxide (CO₂) incubator.

The used medium was removed. Cells in the LPS control group wells, platelet plasma experimental group wells, and purified platelet experimental group wells were suspended in DMEM containing 2% FBS and 5 µg/mL LPS. Cells in the untreated wells were suspended in DMEM containing 2% FBS without LPS. The cells were incubated for 24 hours in a 37°C, 5% carbon dioxide (CO₂) incubator. Then, the following steps were performed separately for each group.

Untreated wells: The used medium was removed. Cells were suspended in DMEM containing 2% FBS and incubated for an additional 24 hours in a 37°C, 5% carbon dioxide (CO₂) incubator.

LPS control group wells: The used medium was removed. Cells in the LPS control wells were suspended in DMEM containing 2% FBS and incubated for an additional 24 hours in a 37°C, 5% carbon dioxide (CO₂) incubator.

Platelet plasma experimental group wells: The platelet plasma group exosome dry powder was reconstituted with 1 mL of DMEM containing 2% FBS to prepare the platelet plasma group's exosome medium. Then, it was mixed with DMEM containing 2% FBS (the volume ratio of the platelet plasma group's exosome medium and DMEM containing 2% FBS was 1:19) to prepare the platelet plasma experimental group's medium. The used medium was removed. Cells in the platelet plasma experimental group wells were suspended using the platelet plasma experimental group's medium and incubated for an additional 24 hours in a 37°C, 5% carbon dioxide (CO₂) incubator.

Purified platelet experimental group wells: The pure platelet group exosome dry powder was reconstituted with 1 mL of DMEM containing 2% FBS to prepare the purified platelet group's exosome medium. Then, it was mixed with DMEM containing 2% FBS (the volume ratio of the purified platelet group's exosome medium and DMEM containing 2% FBS was 1:19) to prepare the purified platelet experimental group's medium. The used medium was removed. Cells in the purified platelet experimental group wells were suspended using the purified platelet experimental group's medium and incubated for an additional 24 hours in a 37°C, 5% carbon dioxide (CO₂) incubator.

Cell culture medium from the untreated wells (control group wells), LPS control group wells, platelet plasma experimental group wells, and purified platelet experimental group wells were transferred into separate microcentrifuge tubes. The concentration of human interleukin-6 (human IL-6, an inflammatory factor) in the cell culture medium was measured using an IL-6 ELISA kit (Human IL-6 ELISA Kit, catalog number: ab178013, brand: Abcam). Analysis was then performed with a multimode microplate spectrophotometer (model: Varioskan LUX, brand: Thermo Scientific) set to an absorbance wavelength of 450 nm. A regression curve of absorbance versus inflammatory factor IL-6 concentration was created based on the instructions of the ELISA kit, and the absorbance values were converted into inflammatory factor IL-6 concentration.

Data were presented as mean ± SD, and statistical analysis was performed using a t-test. Groups marked with "#" indicated a statistically significant difference compared with the platelet plasma experimental group (p < 0.01) while groups marked with "*" indicated a statistically significant difference compared with the LPS control group (p < 0.05).

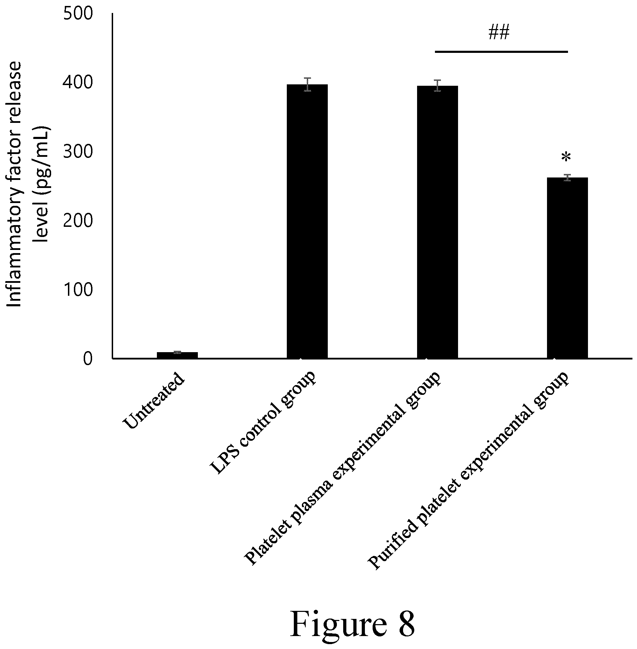

Please refer to Figure 8 for experimental results. Figure 8 is the bar chart of the effects of exosomes in different biological solutions (platelets and plasma) on alleviating inflammation in human dermal fibroblasts.

Figure 8 shows that the average inflammatory factor release level was 9 pg/mL in the untreated group. The average inflammatory factor release level was 396.7 pg/mL in the LPS control group. The average inflammatory factor release level was 394.9 pg/mL in the platelet plasma experimental group. The average inflammatory factor release level was 261.7 pg/mL in the purified platelet experimental group. Among these, there was a significant difference in inflammatory factor release level between the purified platelet experimental group and the LPS control group (p < 0.05). In addition, a significant difference was also observed between the purified platelet experimental group and the platelet plasma experimental group (p < 0.01). It could be seen that high-purity platelet-derived exosome compositions (i.e., removing exosomes derived from cells other than platelets) could effectively alleviate the inflammation, demonstrating an unexpected effect.

### Experiment 8: Effects of a freeze-drying process on the anti-inflammatory efficacy of the product

A purified platelet-derived exosome solution was prepared following steps (S11')-(S13') in Experiment 1.

HDFs required for this experiment were prepared following the experimental steps of Experiment 6-1. A 96-well plate was divided into untreated wells, LPS control group wells, non-freeze-drying group wells, and freeze-drying group wells, with two replicates per group. HDFs were added to the untreated wells, LPS control group wells, non-freeze-drying group wells, and freeze-drying group wells, ensuring an equal cell number each well. The cells were incubated for 24 hours in a 37°C, 5% carbon dioxide (CO₂) incubator.

The used medium was removed. Cells in the LPS control group wells, non-freeze-drying group wells, and freeze-drying group wells were suspended in DMEM containing 2% FBS and 5 µg/mL LPS. On the other hand, cells in the untreated wells were suspended in DMEM containing 2% FBS without LPS. The cells were incubated for 1 hour in a 37°C, 5% carbon dioxide (CO₂) incubator. Then, the following steps were performed separately for each group.

Untreated wells: The used medium was removed. DMEM containing 2% FBS was added to the untreated wells. Cells were incubated for an additional 24 hours in a 37°C, 5% carbon dioxide (CO₂) incubator.

LPS control group wells: The used medium was removed. DMEM containing 2% FBS was added to the LPS control group wells. Cells were incubated for an additional 24 hours in a 37°C, 5% carbon dioxide (CO₂) incubator.

Non-freeze-drying group wells: The used medium was removed. 1 mL of purified platelet-derived exosome solution was taken and mixed with DMEM containing 2% FBS (the volume ratio of the purified platelet-derived exosome solution and DMEM containing 2% FBS was 1:19) to obtain a purified platelet-derived exosome medium. The purified platelet-derived exosome medium was then added to the non-freeze-drying group wells. Cells were incubated for an additional 24 hours in a 37°C, 5% carbon dioxide (CO₂) incubator.

Freeze-drying group wells: The used medium was removed. 1 mL of purified platelet-derived exosome solution was taken, and step (S14') of Experiment 1 was performed to prepare the purified platelet-derived exosome dry powder. The purified platelet-derived exosome dry powder was reconstituted with isotonic sodium chloride solution injection to prepare 1 mL of the purified platelet-derived exosome dry powder solution. The 1 mL of the purified platelet-derived exosome dry powder solution was mixed with DMEM containing 2% FBS (the volume ratio of the purified platelet-derived exosome dry powder solution and DMEM containing 2% FBS was 1:19) to obtain a purified platelet-derived exosome dry powder medium. The purified platelet-derived exosome dry powder medium was added to the freeze-drying group wells. Cells were incubated for an additional 24 hours in a 37°C, 5% carbon dioxide (CO₂) incubator.

Cell culture medium from the untreated wells, LPS control group wells, non-freeze-drying group wells, and freeze-drying group wells were transferred into separate microcentrifuge tubes. The concentration of human interleukin-6 (human IL-6, an inflammatory factor) in the cell culture medium was measured using an IL-6 ELISA kit (Human IL-6 ELISA Kit, catalog number: ab178013, brand: Abcam). Analysis was then performed with a multimode microplate spectrophotometer (model: Varioskan LUX, brand: Thermo Scientific) set to an absorbance wavelength of 450 nm. A regression curve of absorbance versus inflammatory factor IL-6 concentration was created based on the instructions of the ELISA kit, and the absorbance values were converted into inflammatory factor IL-6 concentration.

Data were presented as mean ± SD, and statistical analysis was performed using a t-test. Groups marked with "*" or "**" indicated a statistically significant difference compared with the LPS control group (p < 0.05 or p < 0.01); groups marked with "##" indicated a statistically significant difference compared with the non-freeze-drying group (p < 0.01).

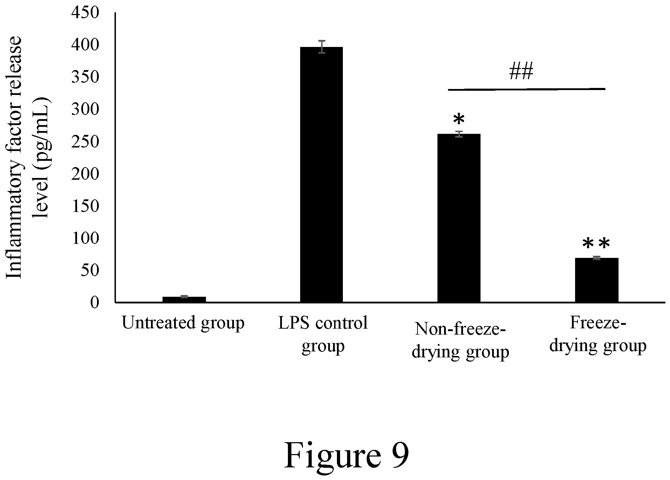

Please refer to Table 6 and Figure 9 for experimental results. Figure 9 is the bar chart of the effect of the freeze-drying process on the anti-inflammatory efficacy of the product.

**Table 6**

| Group | Average inflammatory factor release level (pg/mL) |
|---|---|
| Untreated group | 9 |
| LPS control group | 396.7 |
| Non-freeze-drying group | 261.7 |
| Freeze-drying group | 69.2 |

The experimental results of Figure 9 show that the average inflammatory factor release level was 9 pg/mL in the untreated group; the average inflammatory factor release level was 396.7 pg/mL in the LPS control group; the average inflammatory factor release level was 261.7 pg/mL in the non-freeze-drying group; the average inflammatory factor release level was 69.2 pg/mL in the freeze-drying group. Compared with the LPS control group, both the non-freeze-drying and freeze-drying groups demonstrated a significant reduction in inflammatory factor release levels (p<0.05 or p<0.01). On the other hand, the inflammatory factor release level in the freeze-drying group significantly decreased to 69.2 pg/mL compared with the inflammatory factor release level in the non-freeze-drying group (261.7 pg/mL) (from 100% to 26%; calculated as 69.2 ÷261.7×100%=26%) (p>0.01). It could be seen from the above experiment that the step for performing freeze-drying in this application was a critical technical feature and yielded an unexpected effect.

The inventor expects that after using stimulants (such as LPS) inducing inflammation in other human tissues, the platelet-derived exosome dry powder obtained through the freeze-drying steps in this application may also suppress these induced inflammatory reactions, thereby alleviating inflammation in these tissues.

### Experiment 9: Comparison of platelet-derived exosomes and mesenchymal stromal/stem cell-derived exosomes on tissue repair

### Experiment 9-1: Comparison of platelet-derived exosomes and mesenchymal stromal/stem cell-derived exosomes on cell migration

Mesenchymal stromal/stem cells (MSCs) supernatant was purified using a tangential flow filtration (TFF) system to obtain an MSC-derived exosome solution. Nanoparticle quantitative analysis was performed using a nanoparticle sizer analyzer. The exosome concentration was then adjusted with isotonic sodium chloride solution injection to achieve an MSC-derived exosome solution comprising 10⁷ exosomes per milliliter. The purified platelet-derived exosome solution prepared in steps (S11')-(S13') of Experiment 1 was taken. A nanoparticle quantitative analysis of the solution was performed using a nanoparticle analyzer. The exosome concentration was then adjusted with isotonic sodium chloride solution injection to achieve a purified platelet-derived exosome solution comprising 10⁷ exosomes per milliliter.

Human dermal fibroblasts (HDFs) identical to those used in Experiment 6-1 were utilized for this experiment. HDFs were cultured in Dulbecco's Modified Eagle Medium (DMEM, Corning 10-013CV) containing 2% fetal bovine serum (FBS), followed by replacing the culture medium with fresh medium for later use.

A 96-well plate with cell culture inserts was divided into untreated wells, freeze-thaw 3-cycle group exosome solution wells, and MSC-derived exosome solution wells, with three replicates per group. HDFs were added to the untreated wells, freeze-thaw 3-cycle group exosome solution wells, and MSC-derived exosome solution wells, ensuring an equal cell number each well. The cells were cultured for 24 hours in a 37°C, 5% carbon dioxide (CO₂) incubator. The used medium and each group's cell culture inserts were removed, and the plate was documented by taking photographs under a microscope. Then, the following steps were performed separately for each group.

Untreated wells: DMEM containing 2% FBS was added, and cells were incubated for an additional 6 hours in a 37°C, 5% carbon dioxide (CO₂) incubator.

Freeze-thaw 3-cycle group exosome solution wells: The purified platelet-derived exosome solution was mixed with DMEM containing 2% FBS (the volume ratio was 1:19) to prepare a purified platelet-derived exosome medium. Then, the purified platelet-derived exosome medium was added to the freeze-thaw 3-cycle group exosome solution wells. Cells were incubated for an additional 6 hours in a 37°C, 5% carbon dioxide (CO₂) incubator.

MSC-derived exosome solution wells: The MSC-derived exosome solution was mixed with DMEM containing 2% FBS (the volume ratio was 1:19) to prepare a mesenchymal stromal/stem cell (MSC)-derived exosome medium. Then, the MSC-derived exosome medium was added to the MSC-derived exosome solution wells. Cells were incubated for an additional 6 hours in a 37°C, 5% carbon dioxide (CO₂) incubator.

Photographs were taken using a microscope to document the results, and each group's cell migration coverage area was calculated.

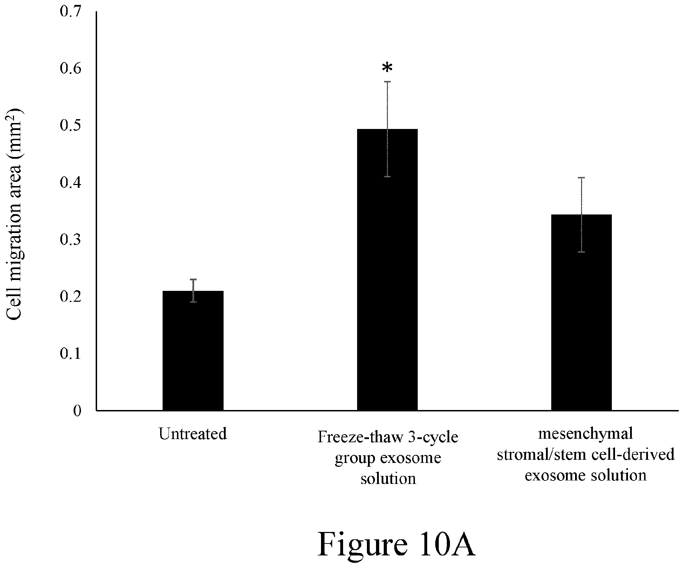

Data were presented as mean ± SD, and statistical analysis was performed using a t-test. Groups marked with "*" indicated a statistically significant difference compared to the MSC-derived exosome solution (p < 0.05). The experimental results are shown in Figure 10A. Figure 10A is the bar chart of the effects of platelet-derived exosomes and mesenchymal stromal/stem cell-derived exosomes on the cell migration of human dermal fibroblasts.

As shown in Figure 10A, the average cell migration coverage area in the untreated group was 0.21 mm²; the average cell migration coverage area in the freeze-thaw 3-cycle group exosome solution group was 0.49 mm²; the average cell migration coverage area in the MSC-derived exosome solution group was 0.34 mm². There was a significant difference between the freeze-thaw 3-cycle group exosome solution group and the MSC-derived exosome solution group (p<0.05). This indicated that the platelet-derived exosome composition in this application exhibited a superior cell migration response compared with the MSC-derived exosomes and was, therefore, more effective in promoting tissue repair.

### Experiment 9-2: Effects of platelet-derived exosomes and MSC-derived exosomes on cell proliferation

A mesenchymal stromal/stem cell (MSC)-derived exosome solution and a purified platelet-derived exosome solution were prepared following the method described in Experiment 9-1. The exosome concentration in the mesenchymal stromal/stem cell (MSC)-derived exosome solution was adjusted to 10⁶ exosomes/mL, and the exosome concentration in the purified platelet-derived exosome solution was adjusted to 10⁶ exosomes/mL

Human dermal fibroblasts (HDFs) identical to those used in Experiment 9-1 were utilized for this experiment. A 96-well plate was divided into untreated wells, freeze-thaw 3-cycle group exosome solution wells, and MSC-derived exosome solution wells, with three replicates per group. HDFs were added to the untreated wells, freeze-thaw 3-cycle group exosome solution wells, and MSC-derived exosome solution wells, ensuring an equal cell number each well. The cells were cultured for 24 hours in a 37°C, 5% carbon dioxide (CO₂) incubator. Then, the following steps were performed separately for each group.

Untreated wells: The used medium was removed. DMEM containing 2% FBS was added to the untreated wells. Cells were incubated for an additional 6 hours in a 37°C, 5% carbon dioxide (CO₂) incubator.

Freeze-thaw 3-cycle group exosome solution wells: The used medium was removed. The purified platelet-derived exosome solution was mixed with DMEM containing 2% FBS (the volume ratio was 1:19) to prepare a purified platelet-derived exosome medium. Then, the purified platelet-derived exosome medium was added to the freeze-thaw 3-cycle group exosome solution wells. Cells were incubated for an additional 6 hours in a 37°C, 5% carbon dioxide (CO₂) incubator.

MSC-derived exosome solution wells: The used medium was removed. The MSC-derived exosome solution was mixed with DMEM containing 2% FBS (the volume ratio was 1:19) to prepare a mesenchymal stromal/stem cell (MSC)-derived exosome medium. Then, the MSC-derived exosome medium was added to the MSC-derived exosome solution wells. Cells were incubated for an additional 6 hours in a 37°C, 5% carbon dioxide (CO₂) incubator.

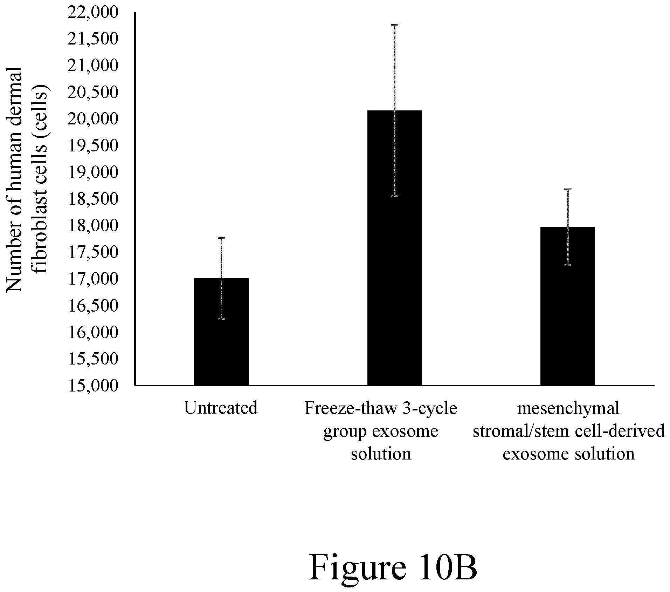

The used medium in each group was removed. 90 µL of DMEM and 10 µL of CCK-8 buffer (DOJINDO, catalog number: CK04) were added to each well. Plates were incubated for an additional 4 hours in a 37°C, 5% carbon dioxide (CO₂) incubator. Analysis was performed with a multimode microplate spectrophotometer (model: Varioskan LUX, brand: Thermo Scientific) set to an absorbance wavelength of 450 nm. A regression curve of absorbance versus cell number was created based on the instructions of the CCK-8 buffer, and absorbance values were converted into viable cell numbers. The experimental results are shown in Figure 10B. Figure 10B is the bar chart of the effects of platelet-derived exosomes and mesenchymal stromal/stem cell-derived exosomes on the cell proliferation of human dermal fibroblasts.

As shown in Figure 10B, the average cell number in the untreated group was 17,010; the average cell number in the freeze-thaw 3-cycle group exosome solution group was 20,157; the average cell number in the MSC-derived exosome solution group was 17,972. In other words, the average cell number in the freeze-thaw 3-cycle group exosome solution group was higher than the average cell number in the MSC-derived exosome solution group. This indicated that the platelet-derived exosome composition in this application had the effect of promoting tissue repair, and the effect was superior to that of MSC-derived exosomes.

### Experiment 9-3: Effects of platelet-derived exosomes and MSC-derived exosomes on collagen expression level

A mesenchymal stromal/stem cell (MSC)-derived exosome solution and a purified platelet-derived exosome solution were prepared following the method described in Experiment 9-1.

Human dermal fibroblasts (HDFs) identical to those used in Experiment 9-1 were utilized for this experiment. A 96-well plate was divided into untreated wells, freeze-thaw 3-cycle group exosome solution wells, and MSC-derived exosome solution wells, with three replicates per group. HDFs were added to the untreated wells, freeze-thaw 3-cycle group exosome solution wells, and MSC-derived exosome solution wells, ensuring an equal cell number each well. The cells were cultured for 24 hours in a 37°C, 5% carbon dioxide (CO₂) incubator. Then, the following steps were performed separately for each group.

Untreated wells: The used medium was removed. DMEM containing 2% FBS was added to the untreated wells. Cells were incubated for an additional 24 hours in a 37°C, 5% carbon dioxide (CO₂) incubator.

Freeze-thaw 3-cycle group exosome solution wells: The used medium was removed. The purified platelet-derived exosome solution was mixed with DMEM containing 2% FBS (the volume ratio was 1:19) to prepare a purified platelet-derived exosome medium. Then, the purified platelet-derived exosome medium was added to the freeze-thaw 3-cycle group exosome solution wells. Cells were incubated for an additional 24 hours in a 37°C, 5% carbon dioxide (CO₂) incubator.

MSC-derived exosome solution wells: The used medium was removed. The MSC-derived exosome solution was mixed with DMEM containing 2% FBS (the volume ratio was 1:19) to prepare a mesenchymal stromal/stem cell (MSC)-derived exosome medium. Then, the MSC-derived exosome medium was added to the MSC-derived exosome solution wells. Cells were incubated for an additional 24 hours in a 37°C, 5% carbon dioxide (CO₂) incubator.

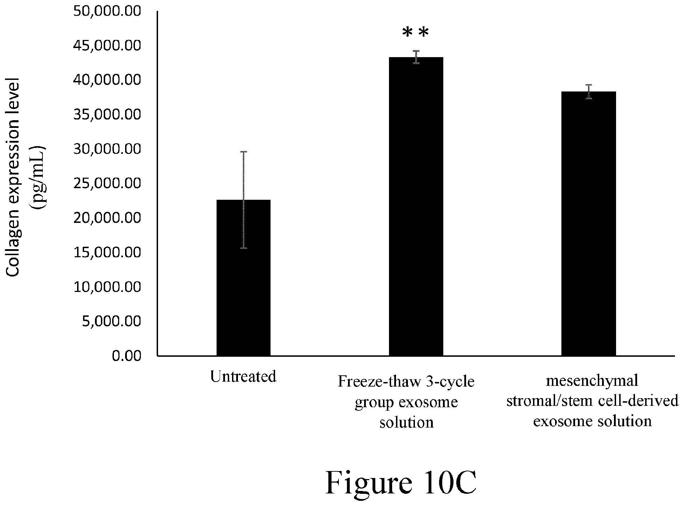

The collagen content in the supernatant of each group was analyzed using an enzyme-linked immunosorbent assay (ELISA). Data were presented as mean ± SD, and statistical analysis was performed using a t-test. Groups marked with "**" indicated a statistically significant difference compared to the MSC-derived exosome solution (p< 0.01). Please refer to Figure 10C for experimental results. Figure 10C is the bar chart of the effects of platelet-derived exosomes and mesenchymal stromal/stem cell-derived exosomes on the collagen expression in human dermal fibroblasts.

As shown in Figure 10C, the average collagen expression level was 22,617.67 pg/mL in the untreated group; the average collagen expression level was 43,311.33 pg/mL in the freeze-thaw 3-cycle group exosome solution group; the average collagen expression level was 38,317.67 pg/mL in the MSC-derived exosome solution group. **In** other words, the average collagen expression level in the freeze-thaw 3-cycle group exosome solution group was higher than the average collagen expression level in the MSC-derived exosome solution group, with a significant difference (p<0.01). This indicated that the platelet-derived exosome composition in this application had a tissue repair-promoting effect, and the effect was superior to that of MSC-derived exosomes.

The above description is only preferred embodiments of the present invention and is not intended to limit the scope of the present invention. Therefore, all other changes or modifications shall be included within the scope of the patent application of the present invention without departing from the spirit of the invention as disclosed herein.

### References

| Serial number | References |
|---|---|
| 1 | Ian A. Darby and Tim D. Hewitson, 2007. Fibroblast Differentiation in Wound Healing and Fibrosis. International Review of Cytology, Vol. 257. |
| 2 | Chadanat Noonin and Visith Thongboonkerd, 2021. Exosome-inflammasome crosstalk and their roles in inflammatory responses. Theranostics 11(9): 4436-4451. |
| 3 | Laura M. Doyle and Michael Zhuo Wang. Overview of Extracellular Vesicles, Their Origin, Composition, Purpose, and Methods for Exosome Isolation and Analysis. Cells 2019, 8, 727. |
| 4 | Yi Zhang et al., Exosome: A Review of Its Classification, Isolation Techniques, Storage, Diagnostic and Targeted Therapy Applications. International Journal of Nanomedicine 2020:15 6917-6934 |

## Claims

1. A method for preparing purified platelet-derived exosome dry powder, comprising:
(a) taking one unit of a solution derived from blood and containing platelets, and performing a process of purifying platelets to obtain a pure platelet solution, wherein step (a) comprises:
(a1) taking one unit of a solution derived from blood and containing platelets;
(a2) performing a first sedimentation process to separate the solution derived from blood and containing platelets into a plasma layer and a blood cell layer;
(a3) removing the blood cell layer to obtain a plasma layer solution;
(a4) performing a second sedimentation process to settle platelets into a pellet;
(a5) removing a supernatant and mixing the pellet with a solvent to obtain the pure platelet solution;
(b) performing an activation procedure to activate platelets in the pure platelet solution and release exosomes to obtain an activated pure platelet solution, wherein the activation procedure is a freezing and then thawing procedure;
(c) performing a purification procedure to obtain a purified platelet-derived exosome solution; and
(d) subjecting the purified platelet-derived exosome solution to a drying process to obtain a purified platelet-derived exosome dry powder containing a plurality of platelet-derived exosomes.

2. The method of claim 1, wherein the drying process is a freeze-drying process.

3. The method of claim 1, wherein step (a3) comprises a process of removing white blood cells so that the number of white blood cells per milliliter (mL) of the pure platelet solution is less than 10⁶.

4. The method of claim 1, wherein step (a5) comprises a process of removing plasma so that albumin concentration in the pure platelet solution is less than 3 g/dL; or step (a5) comprises a process of removing plasma so that globulin concentration in the pure platelet solution is less than 2 g/dL; step (a5) comprises a process of removing plasma so that fibrinogen concentration in the pure platelet solution is less than 100 µg/mL.

5. The method of claim 1, wherein the solution derived from blood and containing platelets is whole blood, and the blood cell layer comprises a buffy coat layer and a red blood cell layer.

6. The method of claim 1, wherein the freezing and then thawing procedure (freeze-thaw procedure) comprises:
(b1') placing a container holding the pure platelet solution in an environment of -70°C ~ - 196°C to freeze the pure platelet solution; and
(b2') raising the temperature of the container to thaw the frozen pure platelet solution; and performing the steps (b1') and (b2') 1, 2, 3, 4, 5, or more than 5 times.

7. The method of claim 1, wherein the freezing and then thawing procedure (freeze-thaw procedure) comprises:
(b1) placing a container holding the pure platelet solution in liquid nitrogen (-196°C) to freeze the pure platelet solution; and
(b2) raising the temperature of the container to thaw the frozen pure platelet solution; and performing the steps (b1) and (b2) 1, 2, 3, 4, 5, or more than 5 times.

8. The method of claim 1, wherein the freezing and then thawing procedure (freeze-thaw procedure) comprises:
(b1') placing a container holding the pure platelet solution in an environment of -80°C to freeze the pure platelet solution; and
(b2') raising the temperature of the container to thaw the frozen pure platelet solution, and performing the steps (b1') and (b2') 1, 2, 3, 4, 5, or more than 5 times.

9. The method of claim 2, wherein the freeze-drying process comprises lowering a temperature to less than or equal to -35°C and lowering a pressure to less than or equal to 80 mTorr.

10. The method of claim 1, wherein the solution derived from blood and containing platelets is whole blood, apheresis platelet, leukocytes-reduced platelets apheresis, or platelet-rich plasma (PRP), or any combination thereof; or
the solution derived from blood and containing platelets is derived from an autologous blood sample or an allogeneic blood sample, or a combination thereof.

11. The method of claim 1, wherein in step (a5), the solvent is sterile saline, water for injection, 0.45% NaCl, 4.5% hypertonic saline, sterile warm spring water, or isotonic saline; or in step (a5), a platelet concentration in the pure platelet solution is 1×10⁹ platelets/mL to 10×10⁹ platelets/mL.

12. The method of claim 1, wherein the purification procedure comprises: (c1) performing a centrifugation process to remove most platelet-associated structures; and (c2) performing a membrane filtration procedure to completely remove platelet-associated structures, wherein the membrane filtration procedure in step (c2) is filtration using a filter membrane with a pore size of less than or equal to 0.45 µm to obtain the purified platelet-derived exosome solution.

13. A purified platelet-derived exosome dry powder prepared by a preparation method, wherein the preparation method comprises:
(a) taking one unit of a solution derived from blood and containing platelets, and performing a process of purifying platelets to obtain a pure platelet solution; wherein step (a) comprises:
(a1) taking one unit of a solution derived from blood and containing platelets;
(a2) performing a first sedimentation process to separate the solution derived from blood and containing platelets into a plasma layer and a blood cell layer;
(a3) removing the blood cell layer to obtain a plasma layer solution;
(a4) performing a second sedimentation process to settle platelets into a pellet;
(a5) removing a supernatant and mixing the pellet with a solvent to obtain the pure platelet solution;
(b) performing an activation procedure to activate platelets in the pure platelet solution and release exosomes to obtain an activated pure platelet solution, wherein the activation procedure is a freezing and then thawing procedure;
(c) performing a purification process to obtain a purified platelet-derived exosome solution; and
(d) subjecting the purified platelet-derived exosome solution to a drying process to obtain a purified platelet-derived exosome dry powder containing a plurality of platelet-derived exosomes.

14. The purified platelet-derived exosome dry powder of claim 13, wherein the drying process is a freeze-drying process.

15. The purified platelet-derived exosome dry powder of claim 13, wherein the freezing and then thawing procedure (freeze-thaw procedure) comprises:
(b1') placing a container holding the pure platelet solution in an environment of -80°C to freeze the pure platelet solution; and
(b2') raising the temperature of the container to thaw the frozen pure platelet solution; and performing the steps (b1') and (b2') 1, 2, 3, 4, 5, or more than 5 times.

16. The purified platelet-derived exosome dry powder of any of claims 13 to 15, wherein in the purified platelet-derived exosome dry powder, a particle diameter of the platelet-derived exosomes is 20-150 nm, and the platelet-derived exosomes comprise an exosome marker and a platelet marker;
wherein the exosome marker is at least one of CD9, CD63, and CD81 or any combination thereof, and the platelet marker is at least one of CD41 and CD42b or a combination thereof; wherein
(1) each gram (g) of the purified platelet-derived exosome dry powder comprises at least 1×10¹⁰ platelet-derived exosomes; or
(2) each gram (g) of the purified platelet-derived exosome dry powder comprises 1×10¹¹-1×10¹⁵ platelet-derived exosomes.

17. The purified platelet-derived exosome dry powder of any of claims 13 to 15, wherein
(1) each gram (g) of the purified platelet-derived exosome dry powder comprises at least 50 µg of microRNA; or
(2) a content of microRNA in each gram (g) of the purified platelet-derived exosome dry powder is 100 µg-2,500 µg.

18. The purified platelet-derived exosome dry powder of any of claims 13 to 15, wherein
(1) a content of PDGF-BB in each gram (g) of the purified platelet-derived exosome dry powder is 0.01 ng-2,000 ng; or
(2) a content of VEGF in each gram (g) of the purified platelet-derived exosome dry powder is 50 pg-100,000 pg; or
(3) a content of IGF in each gram (g) of the purified platelet-derived exosome dry powder is 1 pg-3,000 pg; or
(4) a content of TGF-β1 in each gram (g) of the purified platelet-derived exosome dry powder is 50 ng-20,000 ng; or
(5) a content of EGF in each gram (g) of the purified platelet-derived exosome dry powder is 0.1 ng-200 ng.

19. The purified platelet-derived exosome dry powder of any of claims 13 to 15, wherein
(1) no white blood cell-derived exosome is detectable in each gram of the purified platelet-derived exosome dry powder; or
(2) in each gram of the purified platelet-derived exosome dry powder, a number of white blood cell-derived exosomes is less than or equal to 1×10¹⁴;
wherein the white blood cell-derived exosome comprises a white blood cell marker; the white blood cell marker is CD45.

20. The purified platelet-derived exosome dry powder of any of claims 13 to 15, wherein
(1) no red blood cell-derived exosome is detectable in each gram of the purified platelet-derived exosome dry powder; or
(2) in each gram of the purified platelet-derived exosome dry powder, a number of red blood cell-derived exosomes is less than or equal to 1×10¹⁴;
wherein the red blood cell-derived exosome comprises a red blood cell marker; the red blood cell marker is at least one of CD235a and Annexin V or a combination thereof.

21. The purified platelet-derived exosome dry powder of any of claims 13 to 15, wherein
(1) no exosome other than the platelet-derived exosome is detectable in each gram of the purified platelet-derived exosome dry powder; or
(2) in each gram of the purified platelet-derived exosome dry powder, a number of exosomes other than the platelet-derived exosomes is less than or equal to 1×10¹⁴.

22. Use of the purified platelet-derived exosome dry powder of any of claims 13 to 15 in the manufacture of a pharmaceutical composition or a health composition for administering an effective dose to a site of an individual to relieve a degree of inflammation or injury of the site of the individual.

23. The use of claim 22, wherein the purified platelet-derived exosome dry power is used in the manufacture of the pharmaceutical composition or the health composition to relieve the degree of inflammation or injury in a respiratory tract region, skin region, muscle region, tendon region, bone region, joint region, or ligament region of the individual.

24. Use of the purified platelet-derived exosome dry powder of any of claims 13 to 15 in the manufacture of a pharmaceutical composition or a health composition to enhance a migration capability of dermal fibroblasts into a skin region.

25. The use of claim 24, wherein the skin region is a skin lesion site.

26. Use of the purified platelet-derived exosome dry powder of any of claims 13 to 15 in the manufacture of a pharmaceutical composition or a health composition to enhance a collagen expression level in dermal fibroblasts.
